# EUROPEAN PATENT APPLICATION

(11) **EP 2 238 984 A1**
(43) Date of publication of application: **13.10.2010**
(21) Application number: 09305300.7
(22) Date of filing: 08.04.2009
(51) Int. Cl.: A61K 38/55, A61P 35/00, A23L 1/314

(54) **Caspasin inhibitor peptides**

(71) Applicant: INSTITUT NATIONAL DE LA RECHERCHE AGRONOMIQUE (INRA), 75338 Paris Cédex 07 (FR)
(72) Inventor: Ouali, Ahmed, 63110, BEAUMONT (FR); Talvas, Jérémie, 63000, CLERMONT FERRAND (FR); Rock, Edmond, 63170, Pérignat-Lès-Sarliève (FR); Bremaud, Laure, 87430, VERNEUIL SUR VIENNE (FR); Pelissier, Patrick, 87110, BOSMIE L'AIGUILLE (FR)
(74) Representative: Jacobson, Claude

(57) **Abstract**

The present invention relates to an isolated caspasin inhibitor peptide which comprises or consists in a fragment of a caspasin reactive centre loop and to the use thereof in the prevention or treatment of cancer and for meat ageing.

## Description

### Field of the invention

The present invention relates to caspasin inhibitor peptides.

### Background of the invention

Cell death is necessary for both the normal development of a multicellular organism during embryogenesis and the maintenance of tissue homeostasis over the whole life. A cell can thus commit suicide by activating a tightly regulated cell death program or apoptosis. The tight regulation of this mechanism is essential to ensure that it is only activated in the required cells at the proper moment. Thus, deregulation of apoptosis is known to be associated with pathologies such as cancers, autoimmune diseases and neurodegenerative disorders (Hengartner (2000) Nature 407:770-776).

At the molecular level, the apoptotic cell death machinery forms a complex cascade of ordered events, controlled by the regulated expression of apoptosis-associated genes and proteins. The key components of this machinery are members of the caspase family. Caspases are cysteine peptidases classified apart from peptidases of the papain family. Fourteen different caspases have been identified so far and have been divided into three groups: initiators, effectors and inflammatory (Philchenkov (2004) J. Cell. Mol. Med. 8:432-444).

Due to their critical role in apoptosis, it has been hypothesized that selective activation of caspases could help to combat cancers and other diseases in which pathogenesis had been ascribed to apoptosis dysfunction. This modulation may be direct or indirect, by inactivating caspase inhibitors.

Naturally occurring caspase inhibitors include IAPs (inhibitors of apoptosis proteins) and a group of serpins referred to as endopins or caspasins.

Serpins are globular proteins rich in β-sheets displaying an external mobile loop at their C-terminal end. This loop is responsible of the high instability of these proteins. It contains the active inhibitory site and is responsible of the serpin specificity towards target proteases. Because of their high instability, serpins tend to mask their loop by insertion into the β-stands of another serpin or between the β-sheets of the same molecule. In this last case, serpins are designed as "latent serpins" since the release of the loop from its binding site is a prerequisite (preliminary step) for the serpin interaction with the target enzyme. Upon interaction with the protease, the scissile bond between the C-terminal loop and the rest of the protein is cleaved. The reactive center loop (RCL) then inserts into the core of the protein, forming an additional β-sheet and transporting in the mean time the bound protease to the distal pole of the serpin. This transport induces the distortion of the protease and its inactivation. It remains then covalently bound to the serpin, unable to complete the peptide bond hydrolysis.

Caspasins, also called endopins, are serpins and use the mechanism described above to inhibit their target proteases. Endopins have been first identified by screening cDNAs encoding antichymotrypsin related proteins in chromaffin cells (Hook et al. (2002) Ann. N. Y. Acad. Sci. 971:426-444). Recent investigations aiming at identifying and sequencing the genes encoding caspasin-like serpins in bovine tissues revealed a complex family of at least 8 genes located within the same cluster in the q24 region of chromosome 21. Corresponding proteins are closely related serpins with high structural homology (Pelissier et al. (2008) BMC Genomics 9:151). Two of them, SerpinA3-1 and SerpinA3-3, first called endopin 1 A and 1 B respectively and subsequently renamed caspasins 1 A and 1 B, have been purified from bovine skeletal muscle and studied in their native form (Tassy et al. (2005) Biochem. J. 388:273-280; Herrera-Mendez et al. (2006) FEBS Lett. 580:3477-3484). These caspasins show a high homology with human serpinA3 and serpinA9 orthologues, which appear to be the closest serpins in the human genome.

The correspondence between the different nomenclatures of caspasins is shown in **Table 1**.

**Table 1: Nomenclature of caspasins**

| **A** | **B** | **C** | **D** |
|---|---|---|---|
| Endopin 1 | Endopin 1A | SerpinA3-1 | Caspasin 1-1 |
| | | SerpinA3-2 | Caspasin 1-2 |
| | Endopin 1B | SerpinA3-3 | Caspasin 1-3 |
| | | SerpinA3-4 | Caspasin 1-4 |
| | | SerpinA3-5 | Caspasin 1-5 |
| | | SerpinA3-6 | Caspasin 1-6 |
| Endopin 2 | | SerpinA3-7 | Caspasin 2-1 |
| | | SerpinA3-8 | Caspasin 2-2 |

| | | | |
|---|---|---|---|
| A: nomenclature from Hwang *et al.* (Hwang et al. (1999) J. Biol. Chem. 274:34164-34173) *B:* nomenclature first used by Tassy *et al* (Tassy et al. (2005) Biochem. J. 388:273-280) C: nomenclature proposed after sequencing the 8 genes encoding 8 serpins D: final nomenclature. | | | |

The mode of inhibition of caspases by IAPs greatly differs from the traditional mechanisms known for cystatins, serpins and other peptidase inhibitors families interacting with their target peptidase in a substrate-enzyme manner. By contrast, binding with relatively low affinity (Kd in the low nM range) of IAPs in the vicinity of the active site creates a sufficient steric obstruction to prevent access of the active site to protein substrates, small peptide substrates being hydrolysed after IAPs binding (Fuentes-Prior and Salvesen (2004) Biochem. J. 384:201-232). The anti-apoptotic function of the IAP proteins family can be cancelled by specific inhibitors. After the initiation of apoptosis, Smac/DIABLO and Omi/HtrA2 proteins are released from mitochondria to the cytosol along with cytochrome c. Both Smac and Omi contain an IAP binding motif (IBM) through which they bind tightly to IAPs (Huang et al. (2003) J. Biol. Chem. 278:49517-49522) and release IAPs bound caspase (Saelens et al. (2004) Oncogene 23:2831-2874). Another potential caspase inhibitor, called proteinase inhibitor 9 (PI9), has been identified in human but its ability to inhibit caspases, including caspases 1, 4, 8 and 3, is too low to be physiologically efficient (Annand et al. (1999) Biochem. J. 342:655-665). Besides IAPs, two viral active-site-directed caspases inhibitors have been described: the serpin CrmA (cytokine response modifier A) from cowpox virus, and the baculovirus inhibitor p35 (Cassens et al. (2003) Arch. Immunol. Ther. Exp. (Warsz) 51:19-27). Both form thioester bonds with the active site cysteine residue. Although qualitatively very different from each other, the induced structural changes affecting CrmA and, to a much lesser extent, p35 inhibitors avoided deacylation of the thioester bond leading to a pseudo-irreversible enzyme/inihibitor complex.

Most of these inhibitors, when bound to the target caspase, are hydrolyzed in inactive fragments which are released and eliminated from the cell. Thus, the inhibitor pool in the cell is quickly exhausted by hydrolysis by target enzymes.

Accordingly, there is a need for new efficient and long-lasting compounds capable of activating caspases for treating diseases related to apoptosis deficiency.

### Summary of the invention

The present invention arises from the design, by the inventors, of peptides, originating from caspasin or serpin reactive centre loops which are able to decrease cell proliferation by inhibiting caspasins.

Thus, the present invention relates to an isolated caspasin inhibitor peptide which comprises or consists in
(i) a caspasin reactive centre loop (RCL) or a fragment thereof of at least 3 continuous amino acids, or
(ii) a sequence which is at least 80% identical to the sequence defined in (i), provided that said sequence inhibits caspasins.

The present invention also relates to an isolated nucleic acid which encodes a caspasin inhibitor peptide as defined herein.

Another object of the present invention is the caspasin inhibitor peptide as defined herein for use as a medicament.

The present invention also relates to an *in vitro* method for inhibiting caspasin in a cell or a tissue comprising contacting said cell or said tissue with a caspasin inhibitor peptide as defined herein.

The present invention also relates to the use of a caspasin inhibitor peptide as defined herein for meat ageing, and to an *in vitro* method for meat ageing comprising contacting a muscle or part of muscle taken from a non-human slaughtered animal with a caspasin inhibitor peptide as defined herein.

### Detailed description of the invention

### Caspasin inhibitor peptides

In the context of the invention, a "caspasin inhibitor peptide" refers to a peptide which is able to inhibit the inhibitory activity of at least one caspasin towards a caspase.

In the context of the invention, the terms "polypeptide" and "peptide" are used indifferently and refer to native peptides (either proteolysis products or synthetically synthesized peptides) and further to peptidomimetics, such as peptoids and semipeptoids which are peptide analogs, which may have, for example, modifications rendering the peptides more stable while in a body, or more immunogenic. Such modifications include, but are not limited to, cyclization, N-terminus modification, C-terminus modification, peptide bond modification, including, but not limited to, CH₂-NH, CH₂-S, CH₂-S=O, O=C-NH, CH₂-O, CH₂-CH₂, S=C-NH, CH=CH or CF=CH, backbone modification and residue modification. Methods for preparing peptidomimetic compounds are well known in the art and are specified in Quantitative Drug Design, CA. Ramsden Gd., Chapter 17.2, F. Choplin Pergamon Press (1992).

According to the invention, a peptide consists of less than 50 amino acids, preferably less than 40 amino acids, more preferably less than 30 amino acids, still preferably less then 20 amino acids. More preferably, peptides according to the invention have a length of from about 3 to about 18 amino acids, from about 3 to about 17 amino acids, from about 3 to about 16 amino acids, from about 3 to about 15 amino acids, from about 3 to about 14 amino acids, from about 3 to about 13 amino acids. Most preferably, peptides according to the invention have a length of 12, 11, 10, 9, 8, 7, 6, 5, 4 or 3 amino acids.

As used herein, the term "amino acid" is understood to include: the 20 naturally occurring amino acids *i.e.* alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine; amino acids harbouring the post-translational modifications which can be found *in vivo* such as hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

As used herein, an isolated peptide is a purified, recombinant, or chemically synthesized peptide.

As used herein, the terms "caspasin", "endopin" and "serpin" are used indifferently. In the context of the invention, caspasins encompass caspasin 1A (bovine SERPINA3-1), caspasin 1 B (bovine SERPINA3-3), bovine SERPINA3-2, bovine SERPINA3-4, bovine SERPINA3-5, bovine SERPINA3-6, bovine SERPINA3-7, bovine SERPINA3-8, human serpin A3, human serpin A9, porcine serpin A31, porcine serpin A32, murine serpin A3N and murine serpin A9.

Preferably, the caspasin is selected from the group consisting in bovine SERPINA3-1, bovine SERPINA3-2, bovine SERPINA3-3, bovine SERPINA3-4, bovine SERPINA3-5, bovine SERPINA3-6, bovine SERPINA3-7, bovine SERPINA3-8, human serpin A3 and human serpin A9. The correspondence between the amino acid sequence of these enzymes and the sequence reference is given in **Table 2.**

**Table 2: Correspondence between caspasins and sequence references**

| **caspasin** | **SEQ ID NO** |
|---|---|
| bovine SERPINA3-1 | 1 |
| bovine SERPINA3-2 | 2 |
| bovine SERPINA3-3 | 3 |
| bovine SERPIN3-4 | 4 |
| bovine SERPINA3-5 | 5 |
| bovine SERPINA3-6 | 6 |
| bovine SERPINA3-7 | 7 |
| bovine SERPINA3-8 | 8 |
| human serpin A3 | 9 |
| human serpin A9 | 10 |
| porcine serpin A31 | 11 |
| porcine serpin A32 | 12 |
| murine serpin A3N | 13 |
| murine serpin A9 | 14 |

Accordingly, preferably, the caspasin comprises or consists in an amino acid sequence selected from SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13 and SEQ ID NO: 14. More preferably, the caspasin comprises or consists in an amino acid sequence selected from SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO: 10. More preferably, the caspasin is selected from bovine SERPINA3-3, human serpin A3 and human serpin A9.

The sequences of the caspasin inhibitor peptides according to the present invention preferably correspond to particular sequences of caspasins located in the reactive centre loop. Without willing to be bound by a particular theory, it is thought that the action of these peptides is based on the property of caspasins of inserting the loop carrying their active site in the sheets of the protein. The peptides according to the invention, when added in excess, occupy the insertion site of the caspasin loop and prevent the loop to insert into this site. Consequently, the caspase is not deformed and proceeds with the catalytic process until release of the native enzyme and of two inactive fragments of caspasin.

As used herein, the "caspasin reactive centre loop" or "RCL" refers to an exposed region of the caspasin which includes the specificity determining region and forms the initial interaction with the target protease. Preferably, the sequence of said caspasin RCL is situated between the sequence EXGTEZ (SEQ ID NO: 15), wherein X is E or T and Z is G or A, and the C-terminal end of said caspasin. More preferably, the sequence of said caspasin RCL is selected from SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28 and SEQ ID NO: 29. Most preferably, the sequence of said caspasin RCL is selected from SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24 and SEQ ID NO: 25.

Accordingly, in a preferred embodiment, the caspasin inhibitor peptide according to the invention comprises or consists in a fragment which consists of
(i) a sequence of at least 3, preferably 4, 5, 6, 7, 8, 9, 10, 11 or 12 continuous amino acids selected from the RCL sequence of said caspasin, or
(ii) a sequence which is at least 80% identical to the sequence defined in (i), provided that said sequence inhibits caspasins.

Preferably, the sequence defined in (i) consists of less than 50 amino acids, preferably less than 40 amino acids, more preferably less than 30 amino acids, still preferably less than 20 amino acids. More preferably, the sequence defined in (i) has a length of from about 3 to about 18 amino acids, from about 3 to about 17 amino acids, from about 3 to about 16 amino acids, from about 3 to about 15 amino acids, from about 3 to about 14 amino acids, from about 3 to about 13 amino acids.

More preferably, the caspasin inhibitor peptide according to the invention comprises or consists in a fragment which consists of
(i) a sequence of at least 3 continuous amino acids selected from SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28 and SEQ ID NO: 29, or
(ii) a sequence which is at least 80% identical to the sequence defined in (i), provided that said sequence inhibits caspasins.

Most preferably, the caspasin inhibitor peptide according to the invention comprises or consists in a fragment which consists of
(i) a sequence of at least 3 continuous amino acids selected from SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24 and SEQ ID NO: 25, or
(ii) a sequence which is at least 80% identical to the sequence defined in (i), provided that said sequence inhibits caspasins.

As explained herein above, caspasins contain at least one cleavage site. As used herein, a "cleavage site" refers to an amino acid position in the sequence of a protein before or after which an enzyme is likely to specifically cleave said protein. In the context of the invention, the cleavage site is a protease cleavage site and preferably a trypsin cleavage site and/or a caspase cleavage site.

In particular, the cleavage site in the caspasin sequence may be a trypsin cleavage site. It is well-known from those skilled in the art that trypsin preferentially cleaves at arginine and lysine in position P1 (wherein amino acid residues in a substrate undergoing cleavage are designated P1, P2, P3, P4 etc. in the N-terminal direction from the cleaved bond, and the residues in the C-terminal direction are designated P1', P2', P3', P4').

The cleavage site in the caspasin sequence may also be a caspase cleavage site. As used herein, the term "caspase" encompasses caspase 1, caspase 2, caspase 3, caspase 4, caspase 5, caspase 6, caspase 7, caspase 8, caspase 9 and caspase 10. Caspases generally cleave proteins after a glutamic acid residue but the amino acid sequence preceeding the cleaved bond is important and contributes to the definition of each caspase specificity. As well-known from the one skilled in the art, a caspase 1 cleavage site corresponds preferably to the sequence YVHDA (SEQ ID NO: 30), EADG (SEQ ID NO: 31), YVAD (SEQ ID NO: 32), YEVDX (SEQ ID NO: 33) or WEHDX (SEQ ID NO: 34) where X is any amino acid but proline, glutamic acid, aspartic acid, glutamine, lysine, and arginine. The caspase 2 cleavage site corresponds preferably to the sequence DVADX (SEQ ID NO: 35) or DEHDX (SEQ ID NO: 36), where X is any amino acid but proline, glutamic acid, aspartic acid, glutamine, lysine and arginine. The caspase 3 cleavage site corresponds preferably to the sequence DMQDX (SEQ ID NO: 37) or DEVDX (SEQ ID NO: 38), where X is any amino acid but proline, glutamic acid, aspartic acid, glutamine, lysine and arginine. The caspase 4 cleavage site corresponds preferably to the sequence LEVDX (SEQ ID NO: 39) or (W/L)EHDX (SEQ ID NO: 40), where X is any amino acid but proline, glutamic acid, aspartic acid, glutamine, lysine and arginine. The caspase 5 cleavage site corresponds preferably to the sequence (W/L)EHDX (SEQ ID NO: 40), where X is any amino acid but proline, glutamic acid, aspartic acid, glutamine, lysine and arginine. The caspase 6 cleavage site corresponds preferably to the sequence VEIDX (SEQ ID NO: 41) or VEHDX (SEQ ID NO: 42), where X is any amino acid but proline, glutamic acid, aspartic acid, glutamine, lysine and arginine. The caspase 7 cleavage site corresponds preferably to the sequence DEVDX (SEQ ID NO: 38) where X is any amino acid but proline, glutamic acid, aspartic acid, glutamine, lysine and arginine. The caspase 8 cleavage site corresponds preferably to the sequence IETDX (SEQ ID NO: 43) or LETDX (SEQ ID NO: 44), where X is any amino acid but proline, glutamic acid, aspartic acid, glutamine, lysine and arginine. The caspase 9 cleavage site corresponds preferably to the sequence LEHDX (SEQ ID NO: 45) where X is any amino acid but proline, glutamic acid, aspartic acid, glutamine, lysine and arginine. The caspase 10 cleavage site corresponds preferably to the sequence IEADX (SEQ ID NO: 46) where X is any amino acid but proline, glutamic acid, aspartic acid, glutamine, lysine and arginine.

Accordingly, in a preferred embodiment, the caspasin inhibitor peptide according to the present invention comprises or consists in a fragment of a caspasin RCL which consists of
(i) a sequence of at least 3 continuous amino acids selected from the sequence of said caspasin RCL extending from the sequence EXGTEZ (SEQ ID NO:15) as defined above and the cleavage site of said caspasin, or
(ii) a sequence which is at least 80% identical to the sequence defined in (i), provided that said sequence inhibits caspasins.

Preferably, the sequences of the caspasin inhibitor peptides as defined above comprises or consists of 3 continuous amino acids selected from the group consisting of (i) the sequence extending from Glu359 to Arg374 or Asp395 of SEQ ID NO: 1, (ii) the sequence extending from Glu359 to Arg374 or Asp395 of SEQ ID NO: 2, (iii) the sequence extending from Glu359 to Arg374 or Asp395 of SEQ ID NO: 3, (iv) the sequence extending from Glu359 to Arg374 or Asp395 of SEQ ID NO: 4, (v) the sequence extending from Glu359 to Arg374 or Asp395 of SEQ ID NO: 5, (vi) the sequence extending from Glu362 to Arg377 or Asp398 of SEQ ID NO: 6, (vii) the sequence extending from Glu363 to Thr378 or Glu401 of SEQ ID NO: 7, (viii) the sequence extending from Glu364 to Thr380 or Asp402 of SEQ ID NO: 8, (ix) the sequence extending from Glu367 to Ser384 or Asp407 of SEQ ID NO: 9, and (x) the sequence extending from Glu361 to Arg377 or Asp403 of SEQ ID NO: 10.

Preferably, said fragment consists of from 3 to 20 consecutive amino acids from the caspasin RCL sequence, more preferably from 3 to 15 consecutive amino acids from the caspasin RCL sequence, still preferably from 3 to 10 consecutive amino acids from the caspasin RCL sequence. Most preferably, said fragment comprises 4, 5, 6, 7, 8, 9 or 10 consecutive amino acids from the caspasin RCL sequence.

In a preferred embodiment, the caspasin inhibitor peptide according to the invention comprises or consists in an amino acid sequence selected from the group consisting in SEQ ID NO: 47, SEQ ID NO: 48, SEQ ID NO: 49 and SEQ ID NO: 50 or an amino acid sequence which is at least 80% identical to SEQ ID NO: 47, SEQ ID NO: 48, SEQ ID NO: 49 or SEQ ID NO: 50, provided that said amino acid sequence inhibits caspasins.

As intended herein, a first amino acid sequence which is at least x% identical to a second amino acid sequence means that x% represents the number of amino acids of the first sequence which are identical to their paired amino acids of the second sequence when the two sequences are optimally aligned pairwise, with respect to the total length of the second peptide sequence. As intended herein, two sequences are said to be optimally aligned pairwise when x is maximal.

Alignment can be performed manually or automatically. Algorithms for automated aligning of peptide sequences are well-known to the man skilled in the art and are for example described in Altschul et al. (1997) Nucleic Acids Res. 25:3389 and implemented by softwares such as the Blast software.

Said amino acid sequence which is at least 80% identical to the sequence defined above (and which will also be called the "derivative amino acid sequence" herein below) may be obtained from the native peptide sequence by mutation, e.g. by insertion, deletion or substitution of at least one amino acid and/or by chemical treatment.

More preferably, the derivative amino acid sequence is at least 85%, 90%, 95%, 98%, 99% identical to the referenced sequences defined above.

Methods for evaluating the capacity of a peptide of inhibiting a caspasin activity, and more particularly for evaluating whether a peptide comprising a derivative amino acid sequence inhibits caspasins, are well-known from those skilled in the art, and include detection of apoptosis or measurement of cell proliferation. Such methods are notably described in "Cell Proliferation and Apoptosis", Hughes and Huseyin, (2003) Bios Scientific Publishers Ltd. Furthermore, a caspasin inhibitory activity will lead to an increase of apoptosis and a decrease of cell proliferation. By way of example, cancers cells are incubated with or without putative caspasin inhibitor peptides, and more particularly, peptides comprising a derivative amino acid sequence as defined above, for a suitable period and under suitable culture conditions. Cell proliferation may then be assessed by quantifying the DNA precurosor bromo-deoxyuridine (BrdU). Typically, the effect of peptides on cell proliferation is determined using the nonradioactive BrdU-based cell proliferation assay (Roche, Basel, Switzerland) according to the manufacturer's instructions. LNCaP prostatic cells are incubated for 24 hours in a 96-well plastic plate that contains 200 µl RPMI 1640 medium complemented with 1.5 g/I sodium carbonate, 4.5 g/I glucose, 10 mM HEPES, 1.0 mM sodium pyruvate, 10% foetal bovine serum (FBS) and antibiotics. Peptides are pre-incubated for 1h30 in 20 µl of FBS at 37°C, then added to 180 µl of RPMI. Medium from plates is released and the medium containing peptides is added to the cells. The cells are then cultured for another 24 hours. BrdU incorporation is then determined by measuring the absorbance at 450 nm on an ELISA plate reader. A peptide that inhibits will thus lead to a decreased proliferation of treated cells compared to untreated control cells.

Apoptosis may be detected by measuring, for example, DNA fragmentation, caspase activity, loss of mitochondrial membrane potential, increased production of reactive oxygen species (ROS), intracellular acidification, chromatin condensation and phosphatidyl serine (PS) externalisation. DNA fragmentation can be measured, for example, with the TUNEL assay (terminal deoxynucleotide transferase dUTP nick end labelling). Commercial versions of the assay are widely available, for example, APO-BrdU™ TUNEL Assay Kit (Invitrogen), APO-DIRECT™ Kit (BD Biosciences Pharmingen) and ApoAlert™ DNA Fragmentation Assay Kit (Clontech, a Takara Bio Company). Caspase activity can be monitored via fluorogenic, chromogenic and luminescent substrates specific for particular caspases. Commercial assay kits are available for at least caspases 1, 2, 3, 6, 7, 8 and 9. (see, for example, Invitrogen, Chemicon, CalBiochem, BioSource International, Biovision). Loss of mitochondrial membrane potential can be measured with fluorescent dyes that differentially accumulate in healthy active mitochondria. One non-limiting example is the MitoTracker Red system from Invitrogen. Production of reactive oxygen species (ROS) can be measured with fluorescent dyes including, for example, H2DCFDA (Invitrogen). Intracellular acidification can be measured with fluorescent or chromogenic dyes. Chromatin condensation can be measured with fluorescent dyes including, for example, Hoechst 33342 or 4',6'-diamidino-2-phenylindole (DAPI). Phosphatidyl serine (PS) levels can be measured at the cell surface. For example, Annexin V has a high affinity for PS. Numerous commercially available assays are suitable to monitor the binding of labelled Annexin V to the cell surface.

Cell proliferation may be measured by counting the number of cells according to the incubation period or by quantifying BrdU incorporation in the cells.

Preferably, a peptide according to the invention that inhibits caspasin is a peptide that induces at least a 1.5 fold decrease of cell proliferation, more preferably at least a 2 fold decrease of cell proliferation, most preferably at least a 2.5 fold decrease of cell proliferation, when said cell proliferation is assessed by quantifying BrdU incorporation as described above.

Most preferably, the caspasin inhibitor peptide as defined above has the amino acid sequence SEQ ID NO: 48 or SEQ ID NO: 49.

### Isolated nucleic acids

As used herein, the term "isolated nucleic acid" refers to both RNA and DNA, including cDNA, genomic DNA, and synthetic DNA. Nucleic acids can have any three-dimensional structure. A nucleic acid can be double-stranded or single-stranded (*i.e*., a sense strand or an antisense strand). Non-limiting examples of nucleic acids include genes, gene fragments, exons, introns, messenger RNA (mRNA), transfer RNA (tRNA), ribosomal RNA, siRNA, micro-RNA, ribozymes, cDNA, recombinant nucleic acids, and branched nucleic acids. A nucleic acid may contain unconventional or modified nucleotides. Isolated nucleic acids according to the invention may be purified or recombinant.

### Medicament

A method of treatment comprising the administration of a therapeutically effective amount of the caspasin inhibitor peptide as defined above is also included in the present invention.

Any suitable method of administration, known from one skilled in the art may be used. In particular, the peptide may be administered for example by the oral route, by inhalation, or by the parenteral route (in particular by intravenous injection). When the parenteral route is selected, the peptide may be in the form of injectable solutions and suspensions, conditioned in ampoules or flasks. The forms for parenteral delivery are conventionally obtained by mixing the peptide according to the invention with buffers, stabilizers, preservatives, solubilizing agents, isotonic agents and slurrying agents. According to known techniques, these mixtures can then be sterilized and conditioned in the form of intravenous injections. One of skill in the art may use organic phosphate salts-based buffers as buffer. Examples of slurrying agents include methylcellulose, acacia and sodium carboxymethylcellulose. Examples of stabilizers include sodium sulphite and sodium metasulphite, and examples of preservatives include sodium p-hydroxybenzoate, sorbic acid, cresol and chlorocresol.

The amount of peptide administered naturally depends on the administration route, the size and/or weight of the individual, and the detection technique used.

As used herein, a "therapeutically effective amount" refers to an amount of a compound that confers a therapeutic effect on the treated subject. The therapeutic effect may be objective (*i.e*., measurable by some test or marker) or subjective (*i.e*., the subject gives an indication of or feels an effect).

In the context of the present invention, an "individual" denotes a human or non-human mammal, such as a rodent (rat, mouse, rabbit), a primate (chimpanzee), a feline (cat), a canine (dog). Preferably, the individual is human.

The invention relates more particularly to the caspasin inhibitor peptide as defined above for use in the prevention or treatment of cancer.

### In vitro method for inhibiting caspasin in a cell or a tissue

As used herein, a "tissue" refers to an ensemble of cells, not necessarily identical, but from the same origin, that together carry out a specific function. Preferably, a tissue according to the invention is an animal tissue. It encompasses connective tissue, muscle tissue, nervous tissue and epithelial tissue. More preferably, the tissue according to the invention is a muscle tissue.

### Meat ageing

As used herein, the term "meat ageing" or "meat conditioning" refers to the process, generally comprising holding carcasses or cuts at refrigeration temperatures, preferably from about 1 °C to about 8°C, for extended periods of time after slaughter and initial chill, which leads to the improvement of the tenderness, juiciness, taste and flavour of the meat. Meat ageing encompasses wet ageing and dry ageing. Dry aging consists in hanging meat in a very clean, temperature and humidity controlled cooler for a period of two to four weeks. During this time, enzymes within the meat break down the muscle and connective tissue making it tender. Moisture is lost from the outer parts of the carcass causing an inedible crust to form which must be trimmed off and discarded. Wet aging occurs when meat and its own juices are vacuum packed in plastic and boxed for distribution, a process limiting exsudation (loss of juice).

The present invention also relates to an *in vitro* method for meat ageing comprising contacting a muscle or part of muscle taken from a non-human slaughtered animal with a caspasin inhibitor peptide as defined above.

As used herein, an animal is a non-human mammal or a non-mammal animal. Preferably, the animal is a non-human mammal. More preferably, the animal is from a meat animal species. Most preferably, the animal is a bovine, porcine or ovine.

The invention will be further illustrated by the following figure and example.

### Brief description of the figure

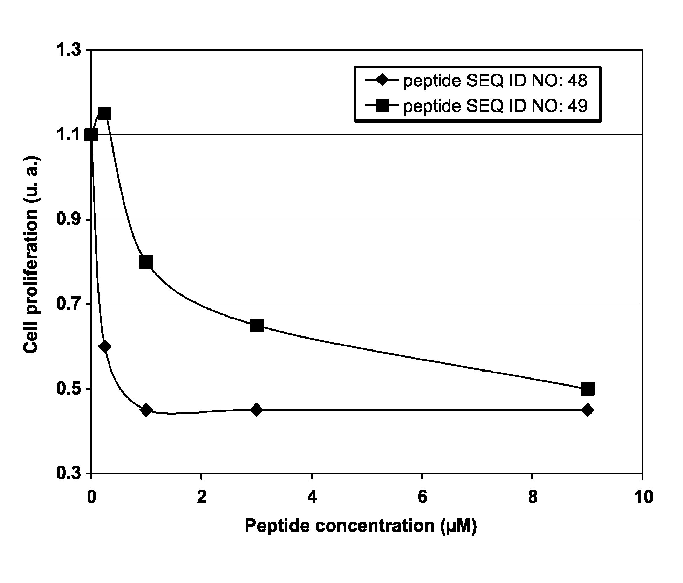

**Figure 1** shows a graph representing the cell proliferation (in arbitrary units) of human prostate cancer cells incubated with different concentrations of peptide of sequence SEQ ID NO: 48 (◆) or of peptide of sequence SEQ ID NO: 49 (■).

### Example

The following example demonstrates the effect of caspasin inhibitor peptides on the proliferation of cancer cells.

### Material and methods

### Peptides and cells

Peptides of sequences SEQ ID NO: 48 and SEQ ID NO: 49 were used in the experiments. They were resuspended in dimethylsulfoxide (DMSO) to obtain a concentration of about 50 mM. They were then diluted in fetal calf serum (FCS) before being added to culture cells at the indicated concentrations.

FGC clone LNCaP human prostate cancer cells, commercialized by ATCC-LGC (Molsheim, France; ATCC Number: CRL-1740) were used.

### Measurement of cell proliferation

The cells were incubated for 48 hours with the caspasin inhibitor peptides at the following concentrations: 0.1, 0.3, 0.9, 3, 10, 20, 30, 60 and 90 µM.

Cell proliferation was then assessed by quantifying the DNA precursor bromo-deoxyuridine (BrdU). This quantification was performed using the 5'-Bromo-2'-deoxy-uridine Labeling and Detection Kit (Roche) according to the manufacturer's instructions.

LNCaP prostatic cells were incubated for 24 hours in a 96-well plastic plate containing 200 µl RPMI 1640 medium complemented with 1.5 g/I sodium carbonate, 4.5 g/I glucose, 10 mM HEPES, 1.0 mM sodium pyruvate, 10% foetal bovine serum (FBS) and antibiotics. Peptides were pre-incubated for 1 h30 in 20 µl of FBS at 37°C, then added to 180 µl of RPMI. Medium from plates was released and the medium containing peptides was added to the cells. The cells were then cultured for another 24 hours. BrdU incorporation was then determined by measuring the absorbance at 450 nm on an ELISA plate reader.

### Results

When LNCaP cells were incubated with the peptides of sequences SEQ ID NO: 48 and SEQ ID NO: 49, a drastic decrease of the cell proliferation was observed at a concentration as lowest as 1 µM (**Figure 1**). A plateau was reached at a concentration of 3 µM of peptide with the peptide of sequence SEQ ID NO: 49.

These results thus demonstrate that the caspasin inhibitor peptides designed by the inventors are capable of decreasing cell proliferation of cancer cells. Accordingly, these peptides can play an important role in the treatment of cancer.

## Claims

1. A caspasin inhibitor peptide which comprises or consists in
(i) a caspasin reactive centre loop (RCL) or a fragment thereof of at least 3 continuous amino acids, or
(ii) a sequence which is at least 80% identical to the sequence defined in (i), provided that said sequence inhibits caspasins.

2. The caspasin inhibitor peptide according to claim 1, wherein the sequence of said caspasin RCL comprises a sequence extending from the sequence EXGTEZ (SEQ ID NO: 15) and the C-terminal end of said caspasin, wherein X is E or T and Z is G or A.

3. The caspasin inhibitor peptide according to claim 1 or 2, wherein the caspasin is selected from the group consisting in bovine SERPINA3-1, bovine SERPINA3-2, bovine SERPINA3-3, bovine SERPINA3-4, bovine SERPINA3-5, bovine SERPINA3-6, bovine SERPINA3-7, bovine SERPINA3-8, human serpin A3, human serpin A9, porcine serpin A31, porcine serpin A32, murine serpin A3N and murine serpin A9.

4. The caspasin inhibitor peptide according to any one of claims 1 to 3, wherein the caspasin comprises or consists in an amino acid sequence selected from SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13 and SEQ ID NO: 14.

5. The caspasin inhibitor peptide according to any one of claims 1 to 4, wherein the caspasin is selected from bovine SERPINA3-3, human serpin A3 and human serpin A9.

6. The caspasin inhibitor peptide according to any one of claims 1 to 4, wherein wherein said fragment consists of
(i) a sequence of at least 3 continuous amino acids selected from SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28 and SEQ ID NO: 29, or
(ii) a sequence which is at least 80% identical to the sequence defined in (i), provided that said sequence inhibits caspasins.

7. The caspasin inhbitor peptide according to any one of claims 1 to 6, which comprises or consists in an amino acid sequence selected from the group consisting in SEQ ID NO: 47, SEQ ID NO: 48, SEQ ID NO: 49 and SEQ ID NO: 50 or an amino acid sequence which is at least 80% identical to SEQ ID NO: 47, SEQ ID NO: 48, SEQ ID NO: 49 or SEQ ID NO: 50, provided that said amino acid sequence inhibits caspasins.

8. The caspasin inhibitor peptide according to claim 7, wherein the amino acid sequence is SEQ ID NO: 48 or SEQ ID NO: 49.

9. An isolated nucleic acid which encodes a caspasin inhibitor peptide according to any one of claims 1 to 8.

10. The caspasin inhibitor peptide according to any one of claims 1 to 8, for use as a medicament.

11. The caspasin inhibitor peptide according to claim 10 for use in the prevention or treatment of cancer.

12. An *in vitro* method for inhibiting caspasin in a cell or a tissue comprising contacting said cell or said tissue with a caspasin inhibitor peptide as defined in any one of claims 1 to 8.

13. The use of a caspasin inhibitor peptide according to any one of claims 1 to 8, for meat ageing.

14. An *in vitro* method for meat ageing comprising contacting a muscle or part of muscle taken from a non-human slaughtered animal with a caspasin inhibitor peptide as defined in any one of claims 1 to 8.


SEQUENCE LISTING

<110>  INRA

<120>  Caspasin inhibitor peptides

<130>  BET 08P0965

<160>  50

<170>  PatentIn version 3.5

<210>  1
<211>  411
<212>  PRT
<213>  Bos taurus


<220>
<221>  MISC_FEATURE
<222>  (1)..(411)
<223>  bovine SERPINA3-1

<220>
<221>  SITE
<222>  (374)..(374)
<223>  trypsin cleavage site

<220>
<221>  SITE
<222>  (395)..(395)
<223>  caspase cleavage site

<400>  1

Met Arg Ala Glu Arg Thr Ser Phe Leu Leu Ala Leu Gly Leu Leu Val
1               5                   10                  15


Ala Gly Ile Arg Ser Val His Cys Leu Pro Glu Asn Val Val Val Lys
            20                  25                  30


Asp Gln His Arg Arg Val Asp Gly His Thr Leu Ala Ser Ser Asn Thr
        35                  40                  45


Asp Phe Ala Phe Ser Leu Tyr Lys Gln Leu Ala Leu Lys Asn Pro Asn
    50                  55                  60


Lys Asn Val Ile Leu Ser Pro Leu Ser Val Ser Ile Ala Leu Ala Phe
65                  70                  75                  80


Leu Ser Leu Gly Ala Arg Gly Ser Thr Leu Thr Glu Ile Leu Glu Gly
                85                  90                  95


Leu Lys Phe Asn Leu Thr Glu Ile Gln Glu Lys Glu Ile His His Ser
            100                 105                 110


Phe Gln His Leu Leu Gln Ala Leu Asn Gln Pro Ser Asn Gln Leu Gln
        115                 120                 125


Leu Ser Val Gly Asn Ala Met Phe Val Gln Glu Glu Leu Lys Leu Leu
    130                 135                 140

Asp Lys Phe Ile Glu Asp Ala Gln Val Leu Tyr Ser Ser Glu Ala Phe
145                 150                 155                 160

Pro Thr Asn Phe Arg Asp Ser Glu Ala Ala Arg Ser Leu Ile Asn Asp
                165                 170                 175

Tyr Val Lys Asn Lys Thr Gln Gly Lys Ile Glu Glu Leu Phe Lys Tyr
            180                 185                 190

Leu Ser Pro Arg Thr Glu Leu Val Leu Val Asn Tyr Ile Tyr Phe Lys
        195                 200                 205

Ala Gln Trp Lys Thr Pro Phe Asp Pro Lys His Thr Glu Gln Ala Glu
    210                 215                 220

Phe His Val Ser Asp Asn Lys Thr Val Glu Val Pro Met Met Thr Leu
225                 230                 235                 240

Asp Leu Glu Thr Pro Tyr Phe Arg Asp Glu Glu Leu Gly Cys Thr Leu
                245                 250                 255

Val Glu Leu Thr Tyr Thr Ser Asn Asp Ser Ala Leu Phe Ile Leu Pro
            260                 265                 270

Asp Glu Gly Lys Met Arg Asp Leu Glu Ala Lys Leu Thr Pro Glu Thr
        275                 280                 285

Leu Thr Arg Trp Arg Asn Ser Leu Gln Pro Arg Arg Ile His Glu Leu
    290                 295                 300

Tyr Leu Pro Lys Phe Ser Ile Lys Ser Asn Tyr Glu Leu Asn Asp Ile
305                 310                 315                 320

Leu Ser Gln Leu Gly Ile Arg Lys Ile Phe Ala Asn Ala Asp Leu Ser
                325                 330                 335

Gly Ile Thr Gly Thr Ala Asp Leu Val Val Ser Gln Val Val His Gly
            340                 345                 350

Ala Ala Leu Asp Val Asp Glu Glu Gly Thr Glu Gly Ala Ala Ala Thr
        355                 360                 365

Gly Ile Ser Met Glu Arg Thr Ile Leu Arg Ile Ile Val Arg Val Asn
    370                 375                 380

Arg Pro Phe Leu Ile Ala Ile Val Leu Lys Asp Thr Gln Ser Ile Ile
385                 390                 395                 400

Phe Leu Gly Lys Val Thr Asn Pro Ser Glu Ala
                405                 410

<210>  2
<211>  411
<212>  PRT
<213>  Bos taurus

<220>
<221>  MISC_FEATURE
<222>  (1)..(411)
<223>  bovine SERPINA3-2

<220>
<221>  SITE
<222>  (374)..(374)
<223>  trypsin cleavage site

<220>
<221>  SITE
<222>  (395)..(395)
<223>  caspase cleavage site

<400>  2

Met Arg Ala Glu Arg Thr Ser Phe Leu Leu Ala Leu Gly Leu Leu Val
1               5                   10                  15

Ala Gly Ile Arg Ser Val His Cys Leu Pro Glu Asn Val Val Val Lys
            20                  25                  30

Asp Gln His Arg Arg Val Asp Gly His Thr Leu Ala Ser Ser Asn Thr
        35                  40                  45

Asp Phe Ala Phe Ser Leu Tyr Lys Gln Leu Ala Leu Lys Asn Pro Asn
    50                  55                  60

Lys Asn Val Ile Leu Ser Pro Leu Ser Val Ser Ile Ala Leu Ala Phe
65                  70                  75                  80

Leu Ser Leu Gly Ala Arg Gly Ser Thr Leu Thr Glu Ile Leu Glu Gly
                85                  90                  95

Leu Lys Phe Asn Leu Thr Glu Ile Gln Glu Lys Glu Ile His His Ser
            100                 105                 110

Phe Gln His Leu Leu Gln Ala Leu Asn Gln Pro Ser Asn Gln Leu Gln
        115                 120                 125

Leu Ser Val Gly Asn Ala Met Phe Val Gln Glu Glu Leu Lys Leu Leu
    130                 135                 140

Asp Lys Phe Ile Glu Asp Ala Gln Val Leu Tyr Ser Ser Glu Ala Phe
145                 150                 155                 160

Pro Thr Asn Phe Arg Asp Ser Glu Ala Ala Arg Ser Leu Ile Asn Asp
                165                 170                 175

Tyr Val Lys Asn Lys Thr Gln Gly Lys Ile Glu Glu Leu Phe Lys Tyr

            180                 185                 190

Leu Ser Pro Arg Thr Glu Leu Val Leu Val Asn Tyr Ile Tyr Phe Lys
        195                 200                 205

Ala Gln Trp Lys Thr Pro Phe Asp Pro Lys His Thr Glu Gln Ala Glu
    210                 215                 220

Phe His Val Ser Asp Asn Lys Thr Val Glu Val Pro Met Met Thr Leu
225                 230                 235                 240

Asp Leu Glu Thr Pro Tyr Phe Arg Asp Glu Glu Leu Gly Cys Thr Leu
                245                 250                 255

Val Glu Leu Thr Tyr Thr Ser Asn Asp Ser Ala Leu Phe Ile Leu Pro
            260                 265                 270

Asp Glu Gly Lys Met Arg Asp Leu Glu Ala Lys Leu Thr Pro Glu Thr
        275                 280                 285

Leu Thr Arg Trp Arg Asn Ser Leu Gln Pro Arg Arg Ile His Glu Leu
    290                 295                 300

Tyr Leu Pro Lys Phe Ser Ile Lys Ser Asn Tyr Glu Leu Asn Asp Ile
305                 310                 315                 320

Leu Ser Gln Leu Gly Ile Arg Lys Ile Phe Ala Asn Ala Asp Leu Ser
                325                 330                 335

Gly Ile Thr Gly Thr Ala Asp Leu Val Val Ser Gln Val Val His Gly
            340                 345                 350

Ala Ala Leu Asp Val Asp Glu Glu Gly Thr Glu Gly Val Ala Ala Thr
        355                 360                 365

Gly Ile Gly Ile Glu Arg Thr Phe Leu Arg Ile Ile Val Arg Val Asn
    370                 375                 380

Arg Pro Phe Leu Ile Ala Val Val Leu Lys Asp Thr Gln Ser Ile Ile
385                 390                 395                 400

Phe Leu Gly Lys Val Thr Asn Pro Ser Glu Ala
                405                 410

<210> 3
<211> 411
<212> PRT
<213> Bos taurus

<220>
<221> MISC_FEATURE
<222> (1)..(411)
<223> bovine SERPINA3-3

<220>
<221>  SITE
<222>  (374)..(374)
<223>  trypsin cleavage site

<220>
<221>  SITE
<222>  (395)..(395)
<223>  caspase cleavage site

<400>  3

Met Arg Ala Glu Arg Leu Ser Pro Leu Leu Ala Leu Gly Leu Leu Val
1               5                   10                  15

Ala Gly Ile Arg Ser Val His Cys Leu Pro Glu Asn Val Val Val Lys
            20                  25                  30

Asp Arg His Arg Arg Val Asp Gly His Thr Leu Ala Ser Ser Asn Thr
        35                  40                  45

Asp Phe Ala Phe Ser Leu Tyr Lys Gln Leu Ala Leu Lys Asn Pro Asn
    50                  55                  60

Lys Asn Val Met Phe Ser Pro Leu Ser Val Ser Met Ala Leu Ala Phe
65                  70                  75                  80

Leu Ser Leu Gly Ala Arg Gly Pro Thr Leu Thr Glu Ile Leu Glu Gly
                85                  90                  95

Leu Lys Phe Asn Leu Thr Glu Ile Gln Glu Thr Gln Ile His Gln Gly
            100                 105                 110

Phe Gln His Leu Leu Gln Ala Leu Asn Arg Pro Arg Asn Gln Leu Gln
        115                 120                 125

Leu Ser Val Gly Asn Ala Met Phe Val Gln Glu Glu Leu Lys Leu Leu
    130                 135                 140

Asp Lys Phe Ile Glu Asp Ala Arg Val Leu Tyr Ser Ser Glu Ala Phe
145                 150                 155                 160

Pro Thr Asn Phe Arg Asp Pro Glu Ala Ala Lys Ser Leu Ile Asn Asp
                165                 170                 175

Tyr Val Lys Asn Lys Thr Gln Gly Lys Ile Glu Glu Leu Phe Lys Asp
            180                 185                 190

Leu Ser Pro Arg Thr Glu Leu Val Leu Val Asn Tyr Val Tyr Phe Lys
        195                 200                 205

Ala Gln Trp Lys Thr Arg Phe Asp Pro Lys His Thr Glu Gln Ala Glu
    210                 215                 220

Phe His Val Ser Asp Asn Lys Thr Val Glu Val Pro Met Met Thr Leu
225                 230                 235                 240

Asp Leu Glu Thr Pro Tyr Phe Arg Asp Glu Glu Leu Gly Cys Thr Leu
                245                 250                 255

Val Glu Leu Thr Tyr Thr Ser Asn Asp Ser Ala Leu Phe Ile Leu Pro
            260                 265                 270

Asp Lys Gly Lys Met Gln Asp Leu Glu Ala Lys Leu Thr Pro Glu Met
        275                 280                 285

Leu Thr Arg Trp Arg Asn Ser Leu Gln Pro Arg Arg Ile His Glu Leu
    290                 295                 300

Tyr Leu Pro Lys Phe Ser Ile Lys Ser Asn Tyr Glu Leu Asn Asp Thr
305                 310                 315                 320

Leu Ser Gln Met Gly Ile Lys Lys Ile Phe Thr Asp Ala Asp Leu Ser
                325                 330                 335

Gly Ile Thr Gly Thr Ala Asp Leu Val Val Ser Gln Val Val His Gly
            340                 345                 350

Ala Ala Leu Asp Val Asp Glu Glu Gly Thr Glu Gly Ala Ala Ala Thr
        355                 360                 365

Gly Ile Gly Ile Glu Arg Thr Phe Leu Arg Ile Ile Val Arg Val Asn
    370                 375                 380

Arg Pro Phe Leu Ile Ala Val Val Leu Lys Asp Thr Gln Ser Ile Ile
385                 390                 395                 400

Phe Leu Gly Lys Val Thr Asn Pro Ser Glu Ala
                405                 410

<210>  4
<211>  411
<212>  PRT
<213>  Bos taurus

<220>
<221>  MISC_FEATURE
<222>  (1)..(411)
<223>  bovine SERPINA3-4

<220>
<221>  SITE
<222>  (374)..(374)
<223>  trypsin cleavage site

<220>
<221>  SITE
<222>  (395)..(395)
<223>  caspase cleavage site

<400> 4

Met Arg Ala Glu Arg Leu Ser Pro Leu Leu Ala Leu Gly Leu Leu Val
1               5                   10                  15

Ala Gly Ile Arg Ser Val His Cys Leu Pro Glu Asn Val Val Val Lys
            20                  25                  30

Asp Arg His Arg Arg Val Asp Gly His Thr Leu Ala Ser Ser Asn Thr
        35                  40                  45

Asp Phe Ala Phe Ser Leu Tyr Lys Gln Leu Ala Leu Lys Asp Pro Asn
    50                  55                  60

Lys Asn Val Met Phe Ser Pro Leu Ser Val Ser Met Ala Leu Ala Phe
65                  70                  75                  80

Leu Ser Leu Gly Ala Arg Gly Pro Thr Leu Thr Glu Ile Leu Glu Gly
                85                  90                  95

Leu Lys Phe Asn Pro Thr Glu Ile Gln Glu Thr Gln Ile His Gln Gly
            100                 105                 110

Phe Gln His Leu Leu Gln Ala Leu Asn Arg Pro Arg Asn Gln Leu Gln
        115                 120                 125

Leu Ser Val Gly Asn Ala Met Phe Val Gln Glu Glu Leu Lys Leu Leu
    130                 135                 140

Asp Lys Phe Ile Glu Asp Ala Arg Val Leu Tyr Ser Ser Glu Ala Phe
145                 150                 155                 160

Pro Thr Asn Phe Arg Asp Pro Glu Ala Ala Lys Ser Leu Ile Asn Asp
                165                 170                 175

Tyr Val Lys Asn Lys Thr Gln Gly Lys Ile Glu Glu Leu Phe Lys Asp
            180                 185                 190

Leu Ser Pro Arg Thr Glu Leu Val Leu Val Asn Tyr Val Tyr Phe Lys
        195                 200                 205

Ala Gln Trp Lys Thr Arg Phe Asp Pro Lys His Thr Glu Gln Ala Glu
    210                 215                 220

Phe His Val Ser Asp Asn Lys Thr Val Glu Val Pro Met Met Thr Leu
225                 230                 235                 240

Asp Leu Glu Thr Pro Tyr Phe Arg Asp Glu Glu Leu Gly Cys Thr Leu
                245                 250                 255

Val Glu Leu Thr Tyr Thr Ser Asn Asp Ser Ala Leu Phe Ile Leu Pro
            260                 265                 270

Asp Lys Gly Lys Met Gln Asp Leu Glu Ala Lys Leu Thr Pro Glu Met
        275                 280                 285

Leu Thr Arg Trp Arg Asn Ser Leu Gln Pro Arg Arg Ile His Glu Leu
    290                 295                 300

Tyr Leu Pro Lys Phe Ser Ile Lys Ser Asn Tyr Glu Leu Asn Asp Thr
305                 310                 315                 320

Leu Ser Gln Met Gly Ile Lys Lys Ile Phe Thr Asp Ala Asp Leu Ser
                325                 330                 335

Gly Ile Thr Gly Thr Ala Asp Leu Val Val Ser Gln Val Val His Gly
            340                 345                 350

Ala Ala Leu Asp Val Asp Glu Glu Gly Thr Glu Gly Ala Ala Ala Thr
        355                 360                 365

Gly Ile Gly Ile Glu Arg Thr Phe Leu Arg Ile Ile Val Arg Val Asn
    370                 375                 380

Arg Pro Phe Leu Ile Ala Val Val Leu Lys Asp Thr Gln Ser Ile Ile
385                 390                 395                 400

Phe Leu Gly Lys Val Thr Asn Pro Ser Glu Ala
                405                 410

<210>  5
<211>  411
<212>  PRT
<213>  Bos taurus

<220>
<221>  MISC_FEATURE
<222>  (1)..(411)
<223>  bovine SERPINA3-5

<220>
<221>  SITE
<222>  (374)..(374)
<223>  trypsin cleavage site

<220>
<221>  SITE
<222>  (395)..(395)
<223>  caspase cleavage site

<400>  5

Met Arg Ala Glu Arg Thr Ser Phe Leu Leu Ala Leu Gly Leu Leu Met
1               5                   10                  15

Ala Gly Ile Arg Ser Val His Cys Leu Pro Glu Asn Val Val Val Lys
            20                  25                  30

Asp Gln Arg Arg Arg Val Asp Ser His Thr Leu Ala Ser Ser Asn Thr

        35                  40                  45

Asp Phe Ala Phe Ser Leu Thr Lys Gln Leu Ala Leu Lys Asn Pro Asn
    50                  55                  60

Lys Asn Val Met Phe Ser Pro Leu Ser Val Ser Met Ala Leu Ala Phe
65                  70                  75                  80

Leu Ser Leu Gly Ala Arg Gly Pro Thr Leu Thr Glu Ile Leu Glu Gly
                85                  90                  95

Leu Lys Phe Asn Leu Thr Glu Ile Gln Glu Thr Gln Ile His Gln Gly
            100                 105                 110

Phe Gln His Leu Leu Gln Ala Leu Asn Arg Pro Ser Asn Gln Leu Gln
        115                 120                 125

Leu Ser Val Gly Asn Ala Met Phe Val Gln Glu Glu Leu Lys Leu Leu
    130                 135                 140

Asp Lys Phe Ile Glu Asp Ala Arg Val Leu Tyr Ser Ser Glu Ala Phe
145                 150                 155                 160

Pro Thr Asn Phe Arg Asp Ser Glu Ala Ala Arg Ser Leu Ile Asn Asp
                165                 170                 175

Tyr Val Lys Asn Lys Thr Gln Gly Lys Ile Glu Glu Leu Phe Lys Tyr
            180                 185                 190

Leu Ser Pro Arg Thr Val Leu Val Leu Val Asn Tyr Ile Tyr Phe Lys
        195                 200                 205

Ala Gln Trp Lys Thr Arg Phe Asp Pro Lys His Thr Glu Gln Ala Glu
    210                 215                 220

Phe His Val Ser Lys Asn Lys Thr Val Glu Val Thr Met Met Thr Leu
225                 230                 235                 240

Asp Leu Glu Thr Pro Tyr Phe Arg Asp Lys Glu Leu Gly Cys Met Leu
                245                 250                 255

Val Glu Leu Thr Tyr Ser Ser Asn Asp Ser Ala Leu Phe Ile Leu Pro
            260                 265                 270

Asp Glu Gly Lys Met Gln Asp Leu Glu Ala Lys Leu Thr Pro Glu Thr
        275                 280                 285

Leu Thr Arg Trp Arg Asn Ser Leu Gln Pro Arg Arg Ile His Glu Leu
    290                 295                 300

Tyr Leu Pro Lys Phe Ser Ile Lys Ser Asn Tyr Glu Leu Asn Asp Thr
305                 310                 315                 320

Leu Ser Gln Met Gly Ile Lys Lys Ile Phe Thr Asp Ala Asp Leu Ser
                325                 330                 335

Gly Ile Thr Gly Thr Ala Asp Leu Val Val Ser Gln Val Val His Gly
            340                 345                 350

Ala Ala Leu Asp Val Asp Glu Glu Gly Thr Glu Gly Ala Ala Ala Thr
        355                 360                 365

Gly Ile Gly Ile Glu Arg Thr Phe Leu Arg Ile Ile Val Arg Val Asn
    370                 375                 380

Arg Pro Phe Leu Ile Ala Val Val Leu Lys Asp Thr Gln Ser Ile Ile
385                 390                 395                 400

Phe Leu Gly Lys Val Thr Asn Pro Ser Glu Ala
                405                 410

<210>  6
<211>  414
<212>  PRT
<213>  Bos taurus

<220>
<221>  MISC_FEATURE
<222>  (1)..(414)
<223>  bovine SERPINA3-6

<220>
<221>  SITE
<222>  (377)..(377)
<223>  trypsin cleavage site

<220>
<221>  SITE
<222>  (398)..(398)
<223>  caspase cleavage site

<400>  6

Met Arg Thr Glu Arg Val Ser Pro Leu Leu Ala Leu Gly Ile Leu Val
1               5                   10                  15

Ala Gly Leu Cys Ser Arg Val His Cys Leu Pro Glu Asn Val Thr Pro
            20                  25                  30

Glu Glu Gln His Lys Val Thr Ser Val Asp Gly His Ser Leu Ala Ser
        35                  40                  45

Ser Asn Thr Asp Phe Ala Phe Ser Leu Tyr Lys Gln Leu Ala Leu Lys
    50                  55                  60

Asp Pro Asn Lys Asn Val Ile Phe Ser Pro Leu Ser Val Ser Ile Ala
65                  70                  75                  80

Leu Ala Phe Leu Ser Leu Gly Ala His Gly Pro Thr Val Thr Glu Ile
                85                  90                  95

Leu Glu Gly Leu Lys Phe Asn Leu Thr Glu Thr Pro Glu Thr Glu Ile
            100                 105                 110

His Gln Gly Phe Gln His Leu Leu Gln Thr Phe Asn Gln Pro Ser Asn
        115                 120                 125

Gln Leu Gln Leu Ser Val Gly Asn Ala Ile Phe Val Gln Glu Glu Leu
    130                 135                 140

Lys Leu Leu Asp Lys Phe Ile Glu Asp Ala Arg Val Leu Tyr Ser Ser
145                 150                 155                 160

Glu Ala Phe Pro Thr Asn Phe Arg Asp Pro Glu Ala Ala Lys Ser Leu
                165                 170                 175

Ile Asn Asp Tyr Val Lys Asn Lys Thr Gln Gly Lys Ile Glu Glu Leu
            180                 185                 190

Phe Lys Asp Leu Ser Pro Arg Thr Glu Leu Val Leu Val Asn Tyr Val
        195                 200                 205

Tyr Phe Lys Ala Gln Trp Lys Thr Arg Phe Asp Pro Lys His Thr Glu
    210                 215                 220

Lys Thr Glu Phe His Val Ser Asp Asn Lys Thr Val Glu Val Pro Met
225                 230                 235                 240

Met Thr Leu Asp Leu Glu Thr Pro Tyr Phe Arg Asp Glu Glu Leu Gly
                245                 250                 255

Cys Thr Leu Val Glu Leu Thr Tyr Thr Ser Asn Asp Ser Ala Leu Phe
            260                 265                 270

Ile Leu Pro Asp Lys Gly Lys Met Gln Asp Leu Glu Ala Lys Leu Thr
        275                 280                 285

Pro Glu Met Leu Thr Arg Trp Arg Asn Ser Leu Gln Pro Arg Arg Ile
    290                 295                 300

His Glu Leu Tyr Leu Pro Lys Phe Ser Ile Lys Ser Asn Tyr Glu Leu
305                 310                 315                 320

Asn Asp Thr Leu Ser Gln Met Gly Ile Lys Lys Ile Phe Thr Ala Asp
                325                 330                 335

Ala Leu Ser Gly Ile Thr Gly Thr Ala Asp Leu Val Val Ser Gln Val
            340                 345                 350

Val His Gly Ala Ala Leu Asp Val Asp Glu Glu Gly Thr Glu Gly Ala

355                 360                 365

Ala Ala Thr Gly Ile Gly Ile Glu Arg Thr Phe Leu Arg Ile Ile Val
    370                 375                 380

Arg Val Asn Arg Pro Phe Leu Ile Ala Val Val Leu Lys Asp Thr Gln
385                 390                 395                 400

Ser Ile Ile Phe Leu Gly Lys Val Thr Asn Pro Ser Glu Ala
                405                 410

<210>  7
<211>  417
<212>  PRT
<213>  Bos taurus

<220>
<221>  MISC_FEATURE
<222>  (1)..(417)
<223>  bovine SERPINA3-7

<220>
<221>  SITE
<222>  (378)..(378)
<223>  trypsin cleavage site

<220>
<221>  SITE
<222>  (401)..(401)
<223>  caspase cleavage site

<400>  7

Met Arg Thr Glu Arg Thr Ser Phe Leu Leu Ala Leu Gly Leu Leu Val
1               5                   10                  15

Ser Gly Phe Cys Ser Arg Val His Cys Leu Pro Glu Asn Val Thr Pro
            20                  25                  30

Glu Glu Gln His Lys Gly Thr Ser Val Asp Gly His Ser Leu Ala Ser
        35                  40                  45

Ser Asn Thr Asp Phe Ala Phe Ser Leu Tyr Lys Gln Leu Ala Leu Lys
    50                  55                  60

Asp Pro Asn Lys Asn Val Ile Phe Ser Pro Leu Ser Ile Ser Ile Ala
65                  70                  75                  80

Leu Ala Phe Leu Ser Leu Gly Ala His Asp His Thr Val Thr Glu Ile
                85                  90                  95

Leu Glu Gly Leu Lys Phe Asn Leu Thr Glu Thr Pro Glu Thr Glu Ile
            100                 105                 110

His Gln Gly Phe Gln His Leu Leu Gln Thr Phe Asn Gln Pro Ser Asn
        115                 120                 125

Gln Leu Gln Leu Ser Val Gly Asn Ala Met Phe Val Ser Glu Glu Leu
    130                 135                 140

Lys Leu Leu Asp Lys Phe Arg Lys Asp Ala Glu Ala Phe Tyr Ala Ser
145                 150                 155                 160

Glu Val Leu Ser Thr Asn Phe Lys Asp Ser Glu Ala Ala Val Lys Leu
                165                 170                 175

Ile Asn Glu Tyr Val Lys Asn Lys Thr His Gly Lys Ile Glu Lys Leu
            180                 185                 190

Phe Asn Asp Leu Asp Val Leu Thr Asn Leu Ile Leu Val Asn Tyr Ile
        195                 200                 205

Phe Phe Lys Ala Gln Trp Lys Thr Pro Phe Asn Pro Asn His Thr Tyr
    210                 215                 220

Glu Ser Glu Phe His Val Ser Gln Asn Glu Arg Val Ile Val Pro Met
225                 230                 235                 240

Met Thr Leu Tyr Leu Glu Thr Pro Tyr Phe Arg Asp Glu Glu Leu Gly
                245                 250                 255

Cys Thr Leu Val Glu Leu Thr Tyr Thr Ser Asn Asp Ser Ala Leu Phe
            260                 265                 270

Ile Leu Pro Asp Glu Gly Lys Met Gln Asp Leu Glu Ala Lys Leu Thr
        275                 280                 285

Pro Glu Thr Leu Thr Arg Trp Arg Asn Ser Leu Gln Pro Arg Leu Ile
    290                 295                 300

His Arg Leu Arg Leu Pro Arg Phe Ser Ile Ser Ser His Tyr Gln Leu
305                 310                 315                 320

Lys Asp Ile Leu Ser Gln Leu Gly Ile Lys Lys Ile Phe Thr Ser Asp
                325                 330                 335

Ala Asp Phe Ser Gly Ile Thr Asp Asp His Lys Leu Ala Val Ser His
            340                 345                 350

Val Ile His Lys Ala Val Leu Asp Val Gly Glu Glu Gly Thr Glu Gly
        355                 360                 365

Ala Ala Val Thr Ala Val Val Met Ala Thr Ser Ser Leu Leu His Thr
    370                 375                 380

Leu Thr Val Ser Phe Asn Arg Pro Phe Leu Leu Ser Ile Phe Cys Lys
385                 390                 395                 400

Glu Thr Gln Ser Ile Ile Phe Leu Gly Lys Val Thr Asn Pro Lys Glu
                405                 410                 415

Ala

<210>  8
<211>  418
<212>  PRT
<213>  Bos taurus

<220>
<221>  MISC_FEATURE
<222>  (1)..(418)
<223>  bovine SERPINA3-8

<220>
<221>  SITE
<222>  (380)..(380)
<223>  trypsin cleavage site

<220>
<221>  SITE
<222>  (402)..(402)
<223>  caspase cleavage site

<400>  8

Met Arg Ala Glu Arg Met Ser Pro Leu Leu Ala Leu Gly Leu Leu Val
1               5                   10                  15

Ser Gly Leu Cys Ser Arg Val His Cys Leu Pro Glu Asn Val Ala Pro
            20                  25                  30

Glu Glu Arg His Lys Gly Thr Ser Val Asp Gly His Ser Leu Ala Ser
        35                  40                  45

Ser Asn Thr Asp Phe Ala Phe Ser Leu Tyr Lys Gln Leu Ala Leu Lys
    50                  55                  60

Asn Pro Asn Lys Asn Val Ile Phe Ser Pro Leu Ser Ile Ser Ile Ala
65                  70                  75                  80

Leu Ala Phe Leu Ser Leu Gly Ala Arg Gly Pro Thr Val Thr Glu Ile
                85                  90                  95

Leu Glu Gly Leu Lys Phe Asn Leu Thr Glu Thr Pro Glu Arg Glu Ile
            100                 105                 110

His Gln Gly Phe Gln His Leu Leu Gln Met Leu Ser Arg Pro Ser Asn
        115                 120                 125

Glu Leu Gln Leu Ser Val Gly Asn Thr Met Phe Val Gln Glu Gln Leu
    130                 135                 140

Lys Leu Leu Asp Lys Phe Arg Glu Asp Ala Leu Ala Leu Tyr Thr Ser
145                 150                 155                 160

Glu Ala Phe Ser Thr Asn Phe Lys Asp Pro Glu Thr Ala Lys Ser Leu
                165                 170                 175

Ile Asn Asp Tyr Val Lys Asn Lys Thr Arg Gly Lys Ile Val Asp Leu
            180                 185                 190

Phe Lys Asp Leu Asp Pro Leu Thr Lys Val Ile Leu Val Asn Tyr Ile
        195                 200                 205

Tyr Phe Lys Ala Gln Trp Arg Thr Pro Phe Asp Pro Lys Gln Thr Tyr
    210                 215                 220

Lys Ser Gln Phe His Val Ser Lys Asn Lys Thr Val Glu Val Pro Met
225                 230                 235                 240

Met Ser Ile Gly Asp Leu Val Thr Pro Tyr Phe Arg Asp Glu Glu Leu
                245                 250                 255

Asp Cys Thr Leu Val Glu Leu Thr Tyr Thr Ser Asn Asp Ser Ala Leu
            260                 265                 270

Phe Ile Leu Pro Asp Glu Gly Lys Met Gln Asp Leu Glu Ala Lys Leu
        275                 280                 285

Ile Pro Glu Met Leu Thr Arg Trp Arg Glu Ser Leu Tyr Pro Arg Gly
    290                 295                 300

Ile His Glu Leu Asn Leu Pro Arg Phe Ser Ile Ala Thr Asp Tyr Lys
305                 310                 315                 320

Leu Lys Asp Ile Leu Ser Gln Leu Glu Ile Lys Lys Val Phe Thr Gln
                325                 330                 335

Glu Ala Asp Leu Ser Gly Ile Thr Asp Asp His Glu Leu Glu Val Ser
            340                 345                 350

Gln Val Val His Lys Ala Val Leu Asp Val Gly Glu Glu Gly Thr Glu
        355                 360                 365

Gly Ala Ala Ala Thr Gly Val Lys Val Gly Ile Thr Ser Ile Asn Asn
    370                 375                 380

His Ile Pro Leu Ser Phe Asn Arg Pro Phe Leu Ile Ala Ile Val Leu
385                 390                 395                 400

Lys Asp Thr Gln Ser Ile Ile Phe Leu Gly Lys Val Thr Asn Pro Ser
                405                 410                 415

Gln Ala

<210>  9
<211>  423
<212>  PRT
<213>  Homo sapiens

<220>
<221>  MISC_FEATURE
<222>  (1)..(423)
<223>  human serpin A3

<220>
<221>  SITE
<222>  (384)..(384)
<223>  trypsin cleavage site

<220>
<221>  SITE
<222>  (407)..(407)
<223>  caspase cleavage site

<400>  9

Met Glu Arg Met Leu Pro Leu Leu Ala Leu Gly Leu Leu Ala Ala Gly
1               5                   10                  15

Phe Cys Pro Ala Val Leu Cys His Pro Asn Ser Pro Leu Asp Glu Glu
            20                  25                  30

Asn Leu Thr Gln Glu Asn Gln Asp Arg Gly Thr His Val Asp Leu Gly
        35                  40                  45

Leu Ala Ser Ala Asn Val Asp Phe Ala Phe Ser Leu Tyr Lys Gln Leu
    50                  55                  60

Val Leu Lys Ala Pro Asp Lys Asn Val Ile Phe Ser Pro Leu Ser Ile
65                  70                  75                  80

Ser Thr Ala Leu Ala Phe Leu Ser Leu Gly Ala His Asn Thr Thr Leu
                85                  90                  95

Thr Glu Ile Leu Lys Gly Leu Lys Phe Asn Leu Thr Glu Thr Ser Glu
            100                 105                 110

Ala Glu Ile His Gln Ser Phe Gln His Leu Leu Arg Thr Leu Asn Gln
        115                 120                 125

Ser Ser Asp Glu Leu Gln Leu Ser Met Gly Asn Ala Met Phe Val Lys
    130                 135                 140

Glu Gln Leu Ser Leu Leu Asp Arg Phe Thr Glu Asp Ala Lys Arg Leu
145                 150                 155                 160

Tyr Gly Ser Glu Ala Phe Ala Thr Asp Phe Gln Asp Ser Ala Ala Ala
                165                 170                 175

Lys Lys Leu Ile Asn Asp Tyr Val Lys Asn Gly Thr Arg Gly Lys Ile

180                 185                 190

Thr Asp Leu Ile Lys Asp Leu Asp Ser Gln Thr Met Met Val Leu Val
        195                 200                 205

Asn Tyr Ile Phe Phe Lys Ala Lys Trp Glu Met Pro Phe Asp Pro Gln
    210                 215                 220

Asp Thr His Gln Ser Arg Phe Tyr Leu Ser Lys Lys Lys Trp Val Met
225                 230                 235                 240

Val Pro Met Met Ser Leu His His Leu Thr Ile Pro Tyr Phe Arg Asp
                245                 250                 255

Glu Glu Leu Ser Cys Thr Val Val Glu Leu Lys Tyr Thr Gly Asn Ala
            260                 265                 270

Ser Ala Leu Phe Ile Leu Pro Asp Gln Asp Lys Met Glu Glu Val Glu
        275                 280                 285

Ala Met Leu Leu Pro Glu Thr Leu Lys Arg Trp Arg Asp Ser Leu Glu
    290                 295                 300

Phe Arg Glu Ile Gly Glu Leu Tyr Leu Pro Lys Phe Ser Ile Ser Arg
305                 310                 315                 320

Asp Tyr Asn Leu Asn Asp Ile Leu Leu Gln Leu Gly Ile Glu Glu Ala
                325                 330                 335

Phe Thr Ser Lys Ala Asp Leu Ser Gly Ile Thr Gly Ala Arg Asn Leu
            340                 345                 350

Ala Val Ser Gln Val Val His Lys Ala Val Leu Asp Val Phe Glu Glu
        355                 360                 365

Gly Thr Glu Ala Ser Ala Ala Thr Ala Val Lys Ile Thr Leu Leu Ser
    370                 375                 380

Ala Leu Val Glu Thr Arg Thr Ile Val Arg Phe Asn Arg Pro Phe Leu
385                 390                 395                 400

Met Ile Ile Val Pro Thr Asp Thr Gln Asn Ile Phe Phe Met Ser Lys
                405                 410                 415

Val Thr Asn Pro Lys Gln Ala
            420

<210>  10
<211>  417
<212>  PRT
<213>  Homo sapiens

<220>
<221>  MISC_FEATURE
<222>  (1)..(417)
<223>  human serpin A9

<220>
<221>  SITE
<222>  (377)..(377)
<223>  trypsin cleavage site

<220>
<221>  SITE
<222>  (403)..(403)
<223>  caspase cleavage site

<400>  10

Met Ala Ser Tyr Leu Tyr Gly Val Leu Phe Ala Val Gly Leu Cys Ala
1               5                   10                  15

Pro Ile Tyr Cys Val Ser Pro Ala Asn Ala Pro Ser Ala Tyr Pro Arg
            20                  25                  30

Pro Ser Ser Thr Lys Ser Thr Pro Ala Ser Gln Val Tyr Ser Leu Asn
        35                  40                  45

Thr Asp Phe Ala Phe Arg Leu Tyr Arg Arg Leu Val Leu Glu Thr Pro
    50                  55                  60

Ser Gln Asn Ile Phe Phe Ser Pro Val Ser Val Ser Thr Ser Leu Ala
65                  70                  75                  80

Met Leu Ser Leu Gly Ala His Ser Val Thr Lys Thr Gln Ile Leu Gln
                85                  90                  95

Gly Leu Gly Phe Asn Leu Thr His Thr Pro Glu Ser Ala Ile His Gln
            100                 105                 110

Gly Phe Gln His Leu Val His Ser Leu Thr Val Pro Ser Lys Asp Leu
        115                 120                 125

Thr Leu Lys Met Gly Ser Ala Leu Phe Val Lys Lys Glu Leu Gln Leu
    130                 135                 140

Gln Ala Asn Phe Leu Gly Asn Val Lys Arg Leu Tyr Glu Ala Glu Val
145                 150                 155                 160

Phe Ser Thr Asp Phe Ser Asn Pro Ser Ile Ala Gln Ala Arg Ile Asn
                165                 170                 175

Ser His Val Lys Lys Lys Thr Gln Gly Lys Val Val Asp Ile Ile Gln
            180                 185                 190

Gly Leu Asp Leu Leu Thr Ala Met Val Leu Val Asn His Ile Phe Phe
        195                 200                 205

Lys Ala Lys Trp Glu Lys Pro Phe His Pro Glu Tyr Thr Arg Lys Asn
    210                 215                 220

Phe Pro Phe Leu Val Gly Glu Gln Val Thr Val His Val Pro Met Met
225                 230                 235                 240

His Gln Lys Glu Gln Phe Ala Phe Gly Val Asp Thr Glu Leu Asn Cys
                245                 250                 255

Phe Val Leu Gln Met Asp Tyr Lys Gly Asp Ala Val Ala Phe Phe Val
            260                 265                 270

Leu Pro Ser Lys Gly Lys Met Arg Gln Leu Glu Gln Ala Leu Ser Ala
        275                 280                 285

Arg Thr Leu Arg Lys Trp Ser His Ser Leu Gln Lys Arg Trp Ile Glu
    290                 295                 300

Val Phe Ile Pro Arg Phe Ser Ile Ser Ala Ser Tyr Asn Leu Glu Thr
305                 310                 315                 320

Ile Leu Pro Lys Met Gly Ile Gln Asn Val Phe Asp Lys Asn Ala Asp
                325                 330                 335

Phe Ser Gly Ile Ala Lys Arg Asp Ser Leu Gln Val Ser Lys Ala Thr
            340                 345                 350

His Lys Ala Val Leu Asp Val Ser Glu Glu Gly Thr Glu Ala Thr Ala
        355                 360                 365

Ala Thr Thr Thr Lys Phe Ile Val Arg Ser Lys Asp Gly Pro Ser Tyr
    370                 375                 380

Phe Thr Val Ser Phe Asn Arg Thr Phe Leu Met Met Ile Thr Asn Lys
385                 390                 395                 400

Ala Thr Asp Gly Ile Leu Phe Leu Gly Lys Val Glu Asn Pro Thr Lys
                405                 410                 415

Ser

<210>  11
<211>  426
<212>  PRT
<213>  Sus scrofa

<220>
<221>  MISC_FEATURE
<222>  (1)..(426)
<223>  porcine serpin A31

<400>  11

Met Arg Ala Glu Gly Met Ser Leu Phe Leu Ala Leu Gly Leu Leu Val
1               5                   10                  15

Ala Gly Leu Cys Ser Arg Val His Cys Val Pro Ala Asp Asp Leu Ala
            20                  25                  30

Ser Lys Ile Val Thr Leu Lys Asp Gln Ile Lys Lys Leu Pro Val His
        35                  40                  45

Asn Thr Ala Val Val Ser Ser Asn Thr Asp Phe Ala Phe Ser Leu Tyr
    50                  55                  60

Lys Gln Leu Ser Leu Thr Asn Arg His Lys Asn Ile Ile Phe Ser Pro
65                  70                  75                  80

Leu Ser Val Ser Met Ala Leu Phe Leu Ser Leu Gly Ala Arg Gly Pro
                85                  90                  95

Thr Leu Thr Glu Leu Leu Lys Ala Ser Ser Ser Cys His Arg Asp Ser
            100                 105                 110

Leu Arg Gln Lys Ser Thr Gln Gly Phe Gln His Leu Arg Gly Arg Ser
        115                 120                 125

Ile Arg Pro Ser Asn Leu Leu Gln Leu Arg Leu Gly Asn Ala Met Phe
    130                 135                 140

Ile Asp Glu Gln Leu Glu Leu Val Asp Lys Phe Val Gln Asp Ala Arg
145                 150                 155                 160

Glu Leu Tyr His Ser Glu Ala Phe Pro Thr Asn Phe Gln Asp Leu Glu
                165                 170                 175

Ala Ala Arg Arg Leu Ile Asn Asp Tyr Val Lys Asn Lys Thr Glu Gly
            180                 185                 190

Lys Ile Val Glu Leu Ile Lys Arg Leu Ile Pro Val Thr Glu Ala Val
        195                 200                 205

Leu Val Asn Tyr Ile Tyr Phe Lys Ala Gln Trp Met Thr His Phe Asp
    210                 215                 220

Pro Asn Leu Thr Thr Glu Ala Asp Phe Tyr Val Ser Lys Asn Arg Thr
225                 230                 235                 240

Val Arg Val Pro Met Met Ala Ile Lys Asn Leu Thr Val Pro Tyr Phe
                245                 250                 255

Arg Asp Glu Glu Leu Ala Cys Thr Val Val Glu Leu Pro Tyr Thr Ser
            260                 265                 270

Asn Asp Ser Thr Leu Phe Ile Leu Pro Asp Asp Gly Arg Met Ala Ala

275                 280                 285

Val Glu Ala Lys Leu Leu Pro Glu Thr Leu Arg Arg Trp Arg Asp Ser
    290                 295                 300

Leu Gln Pro Arg Arg Ile Glu Ala Leu His Leu Pro Arg Phe Ser Ile
305                 310                 315                 320

Ser Ser Asp Tyr Lys Leu Gln Glu Ile Leu Pro Arg Leu Gly Ile Gln
                325                 330                 335

Lys Ile Phe Ser Glu Glu Ala Asp Leu Ser Gly Ile Thr Asp Thr Thr
            340                 345                 350

Pro Leu Cys Val Ser Gln Val Val His Ser Ala Val Leu Asp Val Asp
        355                 360                 365

Glu Glu Gly Thr Glu Gly Ala Ala Ser Thr Gly Val Val Ile Glu Arg
    370                 375                 380

Lys Ser Phe Glu Asn Phe Ile Val Arg Phe Asp Ser Pro Phe Leu Phe
385                 390                 395                 400

Ala Arg Ser Ser Glu Thr Pro Arg Ala Ser Ser Phe Trp Ala Lys Ser
                405                 410                 415

Ser Thr Pro Ile Gln Pro Arg Ala Pro Leu
            420                 425

<210>  12
<211>  415
<212>  PRT
<213>  Sus scrofa

<220>
<221>  MISC_FEATURE
<222>  (1)..(415)
<223>  porcine serpin A32

<400>  12

Met Ser Leu Phe Leu Ala Leu Gly Leu Leu Val Ala Gly Leu Cys Ser
1               5                   10                  15

Arg Val His Cys Val Pro Ala Asp Asp Pro Ala Ser Lys Ile Val Thr
            20                  25                  30

Leu Lys Asp Gln Ile Lys Lys Leu Pro Ala His Asn Thr Ala Val Val
        35                  40                  45

Ser Ser Asn Thr Asp Phe Ala Phe Ser Leu Tyr Lys Gln Leu Ala Leu
    50                  55                  60

Thr Asn Pro His Glu Asn Val Ile Phe Ser Pro Leu Ser Val Ser Met

65                  70                  75                  80

Ala Leu Ala Phe Leu Ser Leu Gly Ala Arg Gly Pro Thr Leu Thr Glu
                85                  90                  95

Leu Leu Glu Gly Leu Lys Phe Asn Leu Thr Lys Thr Pro Glu Ala Glu
            100                 105                 110

Ile His Gln Gly Phe Gln His Leu Leu Ser Thr Leu Asp Arg Ser Ser
        115                 120                 125

Asn Leu Leu Gln Leu Arg Leu Gly Asn Ala Met Phe Ile Asp Glu Gln
    130                 135                 140

Leu Glu Leu Leu Asp Lys Phe Val Gln Asp Ala His Glu Leu Tyr His
145                 150                 155                 160

Ser Glu Ala Phe Pro Thr Asn Phe Gln Asp Leu Glu Ala Ala Arg Arg
                165                 170                 175

Leu Ile Asn Asp Tyr Val Lys Asn Lys Thr Glu Gly Lys Ile Val Asp
            180                 185                 190

Leu Phe Lys Lys Leu Asp Pro Leu Thr Lys Val Val Leu Val Asn Tyr
        195                 200                 205

Ile Tyr Phe Lys Ala Lys Trp Lys Thr Pro Phe Asn Pro Asn Leu Thr
    210                 215                 220

Thr Glu Ala Asp Phe His Val Ser Lys Asn Arg Thr Val Arg Val Pro
225                 230                 235                 240

Met Met Gly Ile Arg Ala Leu Thr Val Pro Tyr Phe Arg Asp Glu Glu
                245                 250                 255

Leu Ala Cys Thr Val Val Glu Leu Pro Tyr Thr Ser Asn Asp Ser Ala
            260                 265                 270

Leu Phe Ile Leu Pro Asp Asp Gly Arg Met Ala Ala Val Glu Ala Lys
        275                 280                 285

Leu Leu Pro Glu Thr Leu Arg Arg Trp Arg Asp Phe Leu Gln Pro Arg
    290                 295                 300

Trp Ile Val Glu Leu Tyr Leu Pro Lys Phe Ser Ile Ser Ser Asp Tyr
305                 310                 315                 320

Arg Leu His Glu Ile Leu Pro Gln Leu Gly Ile Glu Glu Ile Phe Gly
                325                 330                 335

Asp Asn Ala Asn Leu Ser Gly Ile Thr Asn Thr Lys Pro Leu Lys Val
            340                 345                 350

Ser Gln Val Val His Ser Ala Val Leu Asp Val Asn Glu Glu Gly Thr
        355                 360                 365

Glu Ala Ala Ala Ala Thr Gly Ile Asp Ile Asn Val Arg Ser Leu Glu
    370                 375                 380

Arg Ile Ala Leu His Phe Asn Arg Pro Phe Leu Phe Val Ile Ile Ser
385                 390                 395                 400

Lys Asp Ile Gln Ser Ile Ile Phe Leu Gly Lys Val Thr Lys Pro
                405                 410                 415

<210>  13
<211>  418
<212>  PRT
<213>  Mus musculus

<220>
<221>  MISC_FEATURE
<222>  (1)..(418)
<223>  murine serpin A3N

<400>  13

Met Ala Phe Ile Ala Ala Leu Gly Leu Leu Met Ala Gly Ile Cys Pro
1               5                   10                  15

Ala Val Leu Cys Phe Pro Asp Gly Thr Leu Gly Met Asp Ala Ala Val
            20                  25                  30

Gln Glu Asp His Asp Asn Gly Thr Gln Leu Asp Ser Leu Thr Leu Ala
        35                  40                  45

Ser Ile Asn Thr Asp Phe Ala Phe Ser Leu Tyr Lys Glu Leu Val Leu
    50                  55                  60

Lys Asn Pro Asp Lys Asn Ile Val Phe Ser Pro Leu Ser Ile Ser Ala
65                  70                  75                  80

Ala Leu Ala Val Met Ser Leu Gly Ala Lys Gly Asn Thr Leu Glu Glu
                85                  90                  95

Ile Leu Glu Gly Leu Lys Phe Asn Leu Thr Glu Thr Ser Glu Ala Asp
            100                 105                 110

Ile His Gln Gly Phe Gly His Leu Leu Gln Arg Leu Asn Gln Pro Lys
        115                 120                 125

Asp Gln Val Gln Ile Ser Thr Gly Ser Ala Leu Phe Ile Glu Lys Arg
    130                 135                 140

Gln Gln Ile Leu Thr Glu Phe Gln Glu Lys Ala Lys Thr Leu Tyr Gln
145                 150                 155                 160

Ala Glu Ala Phe Thr Ala Asp Phe Gln Gln Pro Arg Gln Ala Lys Lys
                165                 170                 175

Leu Ile Asn Asp Tyr Val Arg Lys Gln Thr Gln Gly Met Ile Lys Glu
            180                 185                 190

Leu Val Ser Asp Leu Asp Lys Arg Thr Leu Met Val Leu Val Asn Tyr
        195                 200                 205

Ile Tyr Phe Lys Ala Lys Trp Lys Val Pro Phe Asp Pro Leu Asp Thr
    210                 215                 220

Phe Lys Ser Glu Phe Tyr Ala Gly Lys Arg Arg Pro Val Ile Val Pro
225                 230                 235                 240

Met Met Ser Met Glu Asp Leu Thr Thr Pro Tyr Phe Arg Asp Glu Glu
                245                 250                 255

Leu Ser Cys Thr Val Val Glu Leu Lys Tyr Thr Gly Asn Ala Ser Ala
            260                 265                 270

Leu Phe Ile Leu Pro Asp Gln Gly Arg Met Gln Gln Val Glu Ala Ser
        275                 280                 285

Leu Gln Pro Glu Thr Leu Arg Lys Trp Lys Asn Ser Leu Lys Pro Arg
    290                 295                 300

Met Ile Asp Glu Leu His Leu Pro Lys Phe Ser Ile Ser Thr Asp Tyr
305                 310                 315                 320

Ser Leu Glu Asp Val Leu Ser Lys Leu Gly Ile Arg Glu Val Phe Ser
                325                 330                 335

Thr Gln Ala Asp Leu Ser Ala Ile Thr Gly Thr Lys Asp Leu Arg Val
            340                 345                 350

Ser Gln Val Val His Lys Ala Val Leu Asp Val Ala Glu Thr Gly Thr
        355                 360                 365

Glu Ala Ala Ala Ala Thr Gly Val Lys Phe Val Pro Met Ser Ala Lys
    370                 375                 380

Leu Tyr Pro Leu Thr Val Tyr Phe Asn Arg Pro Phe Leu Ile Met Ile
385                 390                 395                 400

Phe Asp Thr Glu Thr Glu Ile Ala Pro Phe Ile Ala Lys Ile Ala Asn
                405                 410                 415

Pro Lys

<210>  14
<211>  418
<212>  PRT
<213>  Mus musculus


<220>
<221>  MISC_FEATURE
<222>  (1)..(418)
<223>  murine serpin A9

<400>  14

Met Gly Ser Ser Ser Phe Tyr Arg Val Leu Leu Leu Val Gly Phe Cys
1               5                   10                  15

Ala Pro Ile Phe Cys Met Leu Ser Ser Asn Pro Tyr Asn Gln Glu Ser
            20                  25                  30

Ser His Leu Pro Ser Met Lys Lys Asn Pro Ala Ser Gln Val Ser Pro
        35                  40                  45

Ser Asn Thr Arg Phe Ser Phe Leu Leu Tyr Gln Arg Leu Ala Gln Glu
    50                  55                  60

Asn Pro Gly Gln Asn Ile Leu Phe Ser Pro Val Ser Ile Ser Thr Ser
65                  70                  75                  80

Leu Ala Met Leu Ser Leu Gly Ala Arg Ser Ala Thr Lys Thr Gln Ile
                85                  90                  95

Leu Arg Thr Leu Gly Phe Asn Phe Thr Trp Val Ser Glu Pro Thr Ile
            100                 105                 110

His Met Gly Phe Glu Tyr Leu Val Arg Ser Leu Asn Lys Cys His Gln
        115                 120                 125

Gly Arg Glu Leu Arg Met Gly Ser Val Leu Phe Ile Arg Lys Glu Leu
    130                 135                 140

Gln Leu Gln Ala Thr Phe Leu Asp Arg Val Lys Lys Leu Tyr Gly Ala
145                 150                 155                 160

Lys Val Phe Ser Glu Asp Phe Ser Asn Ala Ala Thr Ala Gln Ala Gln
                165                 170                 175

Ile Asn Ser Tyr Val Glu Lys Glu Thr Lys Gly Lys Val Val Asp Val
            180                 185                 190

Ile Gln Asp Leu Asp Ser Gln Thr Ala Met Val Leu Val Asn His Ile
        195                 200                 205

Phe Phe Lys Ala Asn Trp Thr Gln Pro Phe Ser Thr Ala Asn Thr Asn
    210                 215                 220

Lys Ser Phe Pro Phe Leu Leu Ser Lys Gly Thr Thr Val His Val Pro
225                 230                 235                 240

Met Met His Gln Thr Glu Ser Phe Ala Phe Gly Val Asp Lys Glu Leu
                245                 250                 255

Gly Cys Ser Ile Leu Gln Met Asp Tyr Arg Gly Asp Ala Val Ala Phe
            260                 265                 270

Phe Val Leu Pro Gly Lys Gly Lys Met Arg Gln Leu Glu Lys Ser Leu
        275                 280                 285

Ser Ala Arg Arg Leu Arg Lys Trp Ser Arg Ser Leu Gln Lys Arg Trp
    290                 295                 300

Ile Lys Val Phe Ile Pro Lys Phe Ser Ile Ser Ala Ser Tyr Asn Leu
305                 310                 315                 320

Glu Thr Ile Leu Pro Lys Met Gly Ile Arg Asp Ala Phe Asn Ser Asn
                325                 330                 335

Ala Asp Phe Ser Gly Ile Thr Lys Thr His Phe Leu Gln Val Ser Lys
            340                 345                 350

Ala Ala His Lys Ala Val Leu Asp Val Ser Glu Glu Gly Thr Glu Ala
        355                 360                 365

Ala Ala Ala Thr Thr Thr Lys Leu Ile Val Arg Ser Arg Asp Thr Pro
    370                 375                 380

Ser Ser Ile Ile Ala Phe Lys Glu Pro Phe Leu Ile Leu Leu Leu Asp
385                 390                 395                 400

Lys Asn Thr Glu Ser Val Leu Phe Leu Gly Lys Val Glu Asn Pro Arg
                405                 410                 415

Lys Met


<210>  15
<211>  6
<212>  PRT
<213>  Artificial Sequence

<220>
<223>  beginning of RCL


<220>
<221>  MISC_FEATURE
<222>  (2)..(2)
<223>  X is E or T

<220>
<221>  MISC_FEATURE

<222>  (6)..(6)
<223>  Z is G or A

<400>  15

Glu Xaa Gly Thr Glu Glx
1               5


<210>  16
<211>  53
<212>  PRT
<213>  Bos taurus


<220>
<221>  MISC_FEATURE
<222>  (1)..(53)
<223>  RCL of bovine SERPINA3-1

<400>  16

Glu Glu Gly Thr Glu Gly Ala Ala Ala Thr Gly Ile Ser Met Glu Arg
1               5                   10                  15


Thr Ile Leu Arg Ile Ile Val Arg Val Asn Arg Pro Phe Leu Ile Ala
            20                  25                  30


Ile Val Leu Lys Asp Thr Gln Ser Ile Ile Phe Leu Gly Lys Val Thr
        35                  40                  45


Asn Pro Ser Glu Ala
    50


<210>  17
<211>  53
<212>  PRT
<213>  Bos taurus


<220>
<221>  MISC_FEATURE
<222>  (1)..(53)
<223>  RCL of bovine SERPINA3-2

<400>  17

Glu Glu Gly Thr Glu Gly Val Ala Ala Thr Gly Ile Gly Ile Glu Arg
1               5                   10                  15


Thr Phe Leu Arg Ile Ile Val Arg Val Asn Arg Pro Phe Leu Ile Ala
            20                  25                  30


Val Val Leu Lys Asp Thr Gln Ser Ile Ile Phe Leu Gly Lys Val Thr
        35                  40                  45


Asn Pro Ser Glu Ala
    50


<210>  18
<211>  53

<212>  PRT
<213>  Bos taurus


<220>
<221>  MISC_FEATURE
<222>  (1)..(53)
<223>  RCL of bovine SERPINA3-3

<400>  18

Glu Glu Gly Thr Glu Gly Ala Ala Ala Thr Gly Ile Gly Ile Glu Arg
1               5                   10                  15


Thr Phe Leu Arg Ile Ile Val Arg Val Asn Arg Pro Phe Leu Ile Ala
            20                  25                  30


Val Val Leu Lys Asp Thr Gln Ser Ile Ile Phe Leu Gly Lys Val Thr
        35                  40                  45


Asn Pro Ser Glu Ala
    50


<210>  19
<211>  53
<212>  PRT
<213>  Bos taurus


<220>
<221>  MISC_FEATURE
<222>  (1)..(53)
<223>  RCL of SERPINA3-4

<400>  19

Glu Glu Gly Thr Glu Gly Ala Ala Ala Thr Gly Ile Gly Ile Glu Arg
1               5                   10                  15


Thr Phe Leu Arg Ile Ile Val Arg Val Asn Arg Pro Phe Leu Ile Ala
            20                  25                  30


Val Val Leu Lys Asp Thr Gln Ser Ile Ile Phe Leu Gly Lys Val Thr
        35                  40                  45


Asn Pro Ser Glu Ala
    50


<210>  20
<211>  53
<212>  PRT
<213>  Bos taurus


<220>
<221>  MISC_FEATURE
<222>  (1)..(53)
<223>  RCL of bovine SERPINA3-5

<400>  20

Glu Glu Gly Thr Glu Gly Ala Ala Ala Thr Gly Ile Gly Ile Glu Arg
1               5                   10                  15

Thr Phe Leu Arg Ile Ile Val Arg Val Asn Arg Pro Phe Leu Ile Ala
            20                  25                  30

Val Val Leu Lys Asp Thr Gln Ser Ile Ile Phe Leu Gly Lys Val Thr
        35                  40                  45

Asn Pro Ser Glu Ala
    50

<210>  21
<211>  53
<212>  PRT
<213>  Bos taurus

<220>
<221>  MISC_FEATURE
<222>  (1)..(53)
<223>  RCL of bovine SERPINA3-6

<400>  21

Glu Glu Gly Thr Glu Gly Ala Ala Ala Thr Gly Ile Gly Ile Glu Arg
1               5                   10                  15

Thr Phe Leu Arg Ile Ile Val Arg Val Asn Arg Pro Phe Leu Ile Ala
            20                  25                  30

Val Val Leu Lys Asp Thr Gln Ser Ile Ile Phe Leu Gly Lys Val Thr
        35                  40                  45

Asn Pro Ser Glu Ala
    50

<210>  22
<211>  55
<212>  PRT
<213>  Bos taurus

<220>
<221>  MISC_FEATURE
<222>  (1)..(55)
<223>  RCL of bovine SERPINA3-7

<400>  22

Glu Glu Gly Thr Glu Gly Ala Ala Val Thr Ala Val Val Met Ala Thr
1               5                   10                  15

Ser Ser Leu Leu His Thr Leu Thr Val Ser Phe Asn Arg Pro Phe Leu
            20                  25                  30

Leu Ser Ile Phe Cys Lys Glu Thr Gln Ser Ile Ile Phe Leu Gly Lys
        35                  40                  45

Val Thr Asn Pro Lys Glu Ala
    50                  55


<210>  23
<211>  55
<212>  PRT
<213>  Bos taurus


<220>
<221>  MISC_FEATURE
<222>  (1)..(55)
<223>  RCL of bovine SERPINA3-8

<400>  23

Glu Glu Gly Thr Glu Gly Ala Ala Ala Thr Gly Val Lys Val Gly Ile
1               5                   10                  15


Thr Ser Ile Asn Asn His Ile Pro Leu Ser Phe Asn Arg Pro Phe Leu
            20                  25                  30


Ile Ala Ile Val Leu Lys Asp Thr Gln Ser Ile Ile Phe Leu Gly Lys
        35                  40                  45


Val Thr Asn Pro Ser Gln Ala
    50                  55


<210>  24
<211>  57
<212>  PRT
<213>  Homo sapiens


<220>
<221>  MISC_FEATURE
<222>  (1)..(57)
<223>  RCL of human serpin A3

<400>  24

Glu Glu Gly Thr Glu Ala Ser Ala Ala Thr Ala Val Lys Ile Thr Leu
1               5                   10                  15


Leu Ser Ala Leu Val Glu Thr Arg Thr Ile Val Arg Phe Asn Arg Pro
            20                  25                  30


Phe Leu Met Ile Ile Val Pro Thr Asp Thr Gln Asn Ile Phe Phe Met
        35                  40                  45


Ser Lys Val Thr Asn Pro Lys Gln Ala
    50                  55


<210>  25
<211>  57
<212>  PRT
<213>  Homo sapiens

<220>
<221>  MISC_FEATURE
<222>  (1)..(57)
<223>  RCL of human serpin A9

<400>  25

Glu Glu Gly Thr Glu Ala Thr Ala Ala Thr Thr Thr Lys Phe Ile Val
1               5                   10                  15

Arg Ser Lys Asp Gly Pro Ser Tyr Phe Thr Val Ser Phe Asn Arg Thr
            20                  25                  30

Phe Leu Met Met Ile Thr Asn Lys Ala Thr Asp Gly Ile Leu Phe Leu
        35                  40                  45

Gly Lys Val Glu Asn Pro Thr Lys Ser
    50                  55

<210>  26
<211>  58
<212>  PRT
<213>  Sus scrofa

<220>
<221>  MISC_FEATURE
<222>  (1)..(58)
<223>  RCL of procine serpin A31

<400>  26

Glu Glu Gly Thr Glu Gly Ala Ala Ser Thr Gly Val Val Ile Glu Arg
1               5                   10                  15

Lys Ser Phe Glu Asn Phe Ile Val Arg Phe Asp Ser Pro Phe Leu Phe
            20                  25                  30

Ala Arg Ser Ser Glu Thr Pro Arg Ala Ser Ser Phe Trp Ala Lys Ser
        35                  40                  45

Ser Thr Pro Ile Gln Pro Arg Ala Pro Leu
    50                  55

<210>  27
<211>  51
<212>  PRT
<213>  Sus scrofa

<220>
<221>  MISC_FEATURE
<222>  (1)..(51)
<223>  RCL of porcine serpin A32

<400>  27

Glu Glu Gly Thr Glu Ala Ala Ala Ala Thr Gly Ile Asp Ile Asn Val
1               5                   10                  15

Arg Ser Leu Glu Arg Ile Ala Leu His Phe Asn Arg Pro Phe Leu Phe
            20                  25                  30

Val Ile Ile Ser Lys Asp Ile Gln Ser Ile Ile Phe Leu Gly Lys Val
        35                  40                  45

Thr Lys Pro
    50

<210>  28
<211>  54
<212>  PRT
<213>  Mus musculus

<220>
<221>  MISC_FEATURE
<222>  (1)..(54)
<223>  RCL of murine serpin A3N

<400>  28

Glu Thr Gly Thr Glu Ala Ala Ala Ala Thr Gly Val Lys Phe Val Pro
1               5                   10                  15

Met Ser Ala Lys Leu Tyr Pro Leu Thr Val Tyr Phe Asn Arg Pro Phe
            20                  25                  30

Leu Ile Met Ile Phe Asp Thr Glu Thr Glu Ile Ala Pro Phe Ile Ala
        35                  40                  45

Lys Ile Ala Asn Pro Lys
    50

<210>  29
<211>  56
<212>  PRT
<213>  Mus musculus

<220>
<221>  MISC_FEATURE
<222>  (1)..(56)
<223>  RCL of murine serpin A9

<400>  29

Glu Glu Gly Thr Glu Ala Ala Ala Ala Thr Thr Thr Lys Leu Ile Val
1               5                   10                  15

Arg Ser Arg Asp Thr Pro Ser Ser Ile Ile Ala Phe Lys Glu Pro Phe
            20                  25                  30

Leu Ile Leu Leu Leu Asp Lys Asn Thr Glu Ser Val Leu Phe Leu Gly
        35                  40                  45

Lys Val Glu Asn Pro Arg Lys Met
    50                  55

<210>  30
<211>  5
<212>  PRT
<213>  Artificial

<220>
<223>  caspase 1 cleavage site 1

<400>  30

Tyr Val His Asp Ala
1               5


<210>  31
<211>  4
<212>  PRT
<213>  Artificial

<220>
<223>  caspase 1 cleavage site 2

<400>  31

Glu Ala Asp Gly
1


<210>  32
<211>  4
<212>  PRT
<213>  Artificial

<220>
<223>  caspase 1 cleavage site 3

<400>  32

Tyr Val Ala Asp
1


<210>  33
<211>  5
<212>  PRT
<213>  Artificial

<220>
<223>  caspase 1 cleavage site 4


<220>
<221>  MISC_FEATURE
<222>  (5)..(5)
<223>  Xaa can be any naturally occurring amino acid but Pro, Glu, Asp,
       Gln, Lys or Arg

<400>  33

Tyr Glu Val Asp Xaa
1               5


<210>  34
<211>  5
<212>  PRT
<213>  Artificial

<220>
<223>  caspase 1 cleavage site 5


<220>
<221>  MISC_FEATURE
<222>  (5)..(5)
<223>  Xaa can be any naturally occurring amino acid but Pro, Glu, Asp,
      Gln, Lys or Arg

<400>  34

Trp Glu His Asp Xaa
1               5


<210>  35
<211>  5
<212>  PRT
<213>  Artificial

<220>
<223>  caspase 2 cleavage site 1


<220>
<221>  MISC_FEATURE
<222>  (5)..(5)
<223>  Xaa can be any naturally occurring amino acid but Pro, Glu, Asp,
      Gln, Lys or Arg

<400>  35

Asp Val Ala Asp Xaa
1               5


<210>  36
<211>  5
<212>  PRT
<213>  Artificial

<220>
<223>  caspase 2 cleavage site 2


<220>
<221>  MISC_FEATURE
<222>  (5)..(5)
<223>  Xaa can be any naturally occurring amino acid but Pro, Glu, Asp,
      Gln, Lys or Arg

<400>  36

Asp Glu His Asp Xaa
1               5


<210>  37
<211>  5
<212>  PRT
<213>  Artificial

<220>
<223>  caspase 3 cleavage site 1


<220>
<221>  MISC_FEATURE

<222>  (5)..(5)
<223>  Xaa can be any naturally occurring amino acid but Pro, Glu, Asp,
       Gln, Lys or Arg

<400>  37

Asp Met Gln Asp Xaa
1               5


<210>  38
<211>  5
<212>  PRT
<213>  Artificial

<220>
<223>  caspase 3 cleavage site 2


<220>
<221>  MISC_FEATURE
<222>  (5)..(5)
<223>  Xaa can be any naturally occurring amino acid but Pro, Glu, Asp,
       Gln, Lys or Arg

<400>  38

Asp Glu Val Asp Xaa
1               5


<210>  39
<211>  5
<212>  PRT
<213>  Artificial

<220>
<223>  caspase 4 cleavage site 1


<220>
<221>  MISC_FEATURE
<222>  (5)..(5)
<223>  Xaa can be any naturally occurring amino acid but Pro, Glu, Asp,
       Gln, Lys or Arg

<400>  39

Leu Glu Val Asp Xaa
1               5


<210>  40
<211>  5
<212>  PRT
<213>  Artificial

<220>
<223>  caspase 4 cleavage site 2


<220>
<221>  MISC_FEATURE
<222>  (1)..(1)
<223>  Xaa can be Trp or Leu

<220>
<221>  MISC_FEATURE
<222>  (5)..(5)

<223>  Xaa can be any naturally occurring amino acid but Pro, Glu, Asp,
       Gln, Lys or Arg

<400>  40

Xaa Glu His Asp Xaa
1               5


<210>  41
<211>  5
<212>  PRT
<213>  Artificial

<220>
<223>  caspase 6 cleavage site 1


<220>
<221>  MISC_FEATURE
<222>  (5)..(5)
<223>  Xaa can be any naturally occurring amino acid but Pro, Glu, Asp,
       Gln, Lys or Arg

<400>  41

Val Glu Ile Asp Xaa
1               5


<210>  42
<211>  5
<212>  PRT
<213>  Artificial

<220>
<223>  caspase 6 cleavage site 2


<220>
<221>  MISC_FEATURE
<222>  (5)..(5)
<223>  Xaa can be any naturally occurring amino acid but Pro, Glu, Asp,
       Gln, Lys or Arg

<400>  42

Val Glu His Asp Xaa
1               5


<210>  43
<211>  5
<212>  PRT
<213>  Artificial

<220>
<223>  caspase 8 cleavage site 1


<220>
<221>  MISC_FEATURE
<222>  (5)..(5)
<223>  Xaa can be any naturally occurring amino acid but Pro, Glu, Asp,
       Gln, Lys or Arg

<400>  43

Ile Glu Thr Asp Xaa

1               5


<210>  44
<211>  5
<212>  PRT
<213>  Artificial

<220>
<223>  caspase 8 cleavage site 2


<220>
<221>  MISC_FEATURE
<222>  (5)..(5)
<223>  Xaa can be any naturally occurring amino acid but Pro, Glu, Asp,
       Gln, Lys or Arg

<400>  44

Leu Glu Thr Asp Xaa
1               5


<210>  45
<211>  5
<212>  PRT
<213>  Artificial

<220>
<223>  caspase 9 cleavage site


<220>
<221>  MISC_FEATURE
<222>  (5)..(5)
<223>  Xaa can be any naturally occurring amino acid but Pro, Glu, Asp,
       Gln, Lys or Arg

<400>  45

Leu Glu His Asp Xaa
1               5


<210>  46
<211>  5
<212>  PRT
<213>  Artificial

<220>
<223>  caspase 10 cleavage site


<220>
<221>  MISC_FEATURE
<222>  (5)..(5)
<223>  Xaa can be any naturally occurring amino acid but Pro, Glu, Asp,
       Gln, Lys or Arg

<400>  46

Ile Glu Ala Asp Xaa
1               5


<210>  47
<211>  7
<212>  PRT

<213>  Artificial

<220>
<223>  peptide 1

<400>  47

Phe Leu Ile Ala Val Val Leu
1               5


<210>  48
<211>  6
<212>  PRT
<213>  Artificial

<220>
<223>  peptide 2

<400>  48

Ala Thr Gly Ile Gly Ile
1               5


<210>  49
<211>  9
<212>  PRT
<213>  Artificial

<220>
<223>  peptide 3

<400>  49

Phe Leu Met Met Ile Thr Asn Lys Ala
1               5


<210>  50
<211>  10
<212>  PRT
<213>  Artificial

<220>
<223>  peptide 4

<400>  50

Asn Arg Pro Phe Leu Met Ile Ile Val Gly
1               5                   10